# EUROPEAN PATENT APPLICATION

(11) **EP 4 825 878 A1**
(43) Date of publication of application: **21.10.2026**
(21) Application number: 24874064.9
(22) Date of filing: 26.09.2024
(51) Int. Cl.: C07K 19/00, C12N 15/62, C12N 15/85, C12N 5/10, A61K 39/205, A61P 31/14, C07K 14/145

(54) **RABIES VIRUS G PROTEIN MUTANT, AND PREPARATION METHOD THEREFOR AND USE THEREOF**

(30) Priority: 15.12.2023 CN 202311726444
(71) Applicant: Yantai Patronus Biotech Co., Ltd., Yantai, Shandong 264670 (CN); Guangzhou Patronus Biotech Co., Ltd., Guangzhou, Guangdong 510000 (CN)
(72) Inventor: ZHANG, Senyan, Guangzhou, Guangdong 510000 (CN); JIN, Jing, Guangzhou, Guangdong 510000 (CN); LI, Yuanyuan, Guangzhou, Guangdong 510000 (CN); TIAN, Siyu, Guangzhou, Guangdong 510000 (CN)
(74) Representative: karo IP
(86) International application number: PCT/CN2024/121316
(87) International publication number: WO 2025/073246

(57) **Abstract**

The present invention relates to the technical field of biomedicine. Provided are a rabies virus G protein mutant, and a preparation method therefor and the use thereof. The rabies virus G protein mutant is obtained by means of a site mutation on the extracellular domain of a G protein. For the mutant, soluble expression of the extracellular domain of the G protein is realized, and the mutant can induce the generation of a neutralizing antibody with a high titer. Compared with commercial rabies vaccines for human and rabies vaccines for dogs, the mutant has better cell immune and antibody immune responses. The mutant is expected to reduce the inoculation frequency, shorten the immunizing procedure, and decrease the immunizing dose.

## Description

### TECHNICAL FIELD

The present disclosure relates to the field of biopharmaceutical technology, and in particular, to a rabies virus G protein mutant, a method for preparing same, and use thereof.

### BACKGROUND

Rabies is a zoonotic disease. It is an acute infectious disease caused by rabies virus (RABV) and mainly attacks the central nervous system, with a mortality rate of almost 100% after development. Rabies virus is an RNA virus of the *Lyssavirus* genus of the *Rhabdoviridae* family. The virus particle is bullet-shaped, 100-300 nm in length, about 75 nm in diameter, and its genome encodes five structural proteins, RNA-dependent RNA polymerase (L protein), nucleoprotein (N protein), phosphoprotein (P protein), matrix protein (M protein), and surface glycoprotein (G protein). The G protein is embedded in the surface of the outer lipid envelope of the rabies virus particle, and a trimer formed by the G protein constitutes a spike. The spike is the site where the virus binds to a host receptor and that contains the major recognition epitopes for neutralizing antibodies. Also, the G protein may be glycosylated, and during its expression and secretion, glycosylation modification plays a crucial role in its stability, antigenicity, and biological activity. Therefore, the G protein is an important target protein for the development of rabies vaccines, antiviral drugs, and antibody diagnostic reagents at present.

The coding region of the gene of the G protein has a length of 1575 nucleotides and encodes 524 amino acids, of which the amino acids at positions 1-19 are a signal peptide. After the signal peptide is cleaved off, a mature protein of 505 amino acids is formed, which comprises three domains: an ectodomain, a transmembrane domain, and an intracellular domain. The ectodomain is a G protein segment exposed on the surface of the virion, a functional segment for receptor recognition and membrane fusion, a truly effective antigen segment and a target for neutralizing antibodies in vaccine preparation. Most eukaryotic expression attempts afforded the full-length rabies virus G protein. Due to the high hydrophobicity of the transmembrane region, the G protein obtained therefrom is very easy to aggregate in a solution to form a highly heterogeneous protein multimer, which is difficult to be effectively purified. It has been reported in the literature that only the G protein ectodomain expressed by the wild-type CVS-11 strain formed irregular aggregates. The rabies virus G protein ectodomain (RABV-G-ecto) was recombinantly engineered to replace the region where amino acids at positions 73-79 are located in the first fusion loop and the region where amino acids at positions 117-125 are located in the second fusion loop of the protein ectodomain by a linker peptide consisting of 5 amino acids (GGSGG), so as to obtain a soluble recombinant RABV-G-ecto protein with uniform properties in solution (see "Structural Analysis of Rabies Virus Glycoprotein Reveals pH-Dependent Conformational Changes and Interactions with a Neutralizing Antibody", Yang et al., Cell Host & Microbe 27,441-453, March 11, 2020; CN109627294B). It has been reported in another document that although the purified rabies virus G protein obtained by only expressing the ectodomain derived from rabies virus G gene (Genbank Accession No. KT221113.1) can produce a high titer of anti-G protein antibodies, it cannot induce the production of neutralizing antibodies in mice, suggesting that the transmembrane region of rabies virus G protein is crucial for the conformation and biological activity of the ectodomain (see "Construction and Identification of Cell Lines Stably Expressing the RABV G Ectodomain in a Secretory Manner", Li Qingqing, Southern Medical University, CNKI, March 1, 2018). It can be seen that it is relatively difficult to obtain a G protein ectodomain that is soluble, has a correct spatial folding, and can induce the production of neutralizing antibodies. There are still many technical difficulties to be overcome when the G protein ectodomain is used to prepare a rabies vaccine.

Also, studies have shown that the conformation of the G protein changes during the process of binding to the receptor and membrane fusion and may switch between the two forms before and after fusion with the host cell. The G protein is in the post-fusion state under an acidic condition and in the pre-fusion state under neutral and alkaline conditions. It is generally believed that the pre-fusion state of the G protein can more effectively induce the production of neutralizing antibodies. In addition, G proteins may also change from a trimeric structure to a monomeric structure. Therefore, this property of the G protein is a challenge and key point in the R&D design of protein vaccines and subsequent process.

At present, rabies vaccines are mainly purified vaccines from the culture of primary hamster kidney cells, chicken embryo cells, human diploid cells, and Vero cells. Among them, despite the advantages of a low incidence of adverse events, mild symptoms, and a good immune effect, the human diploid cell vaccines have the disadvantages of slow cell proliferation, low virus yield, and high vaccine cost, and thus have not yet been widely used. In addition to conventional rabies vaccines, new vaccines are also being rapidly investigated, such as recombinant protein-based vaccines or mRNA vaccines. Moreover, since the current rabies vaccines for human use are all based on the immunization procedure with 4 or 5 injections, the immunization time is relatively long, leading to a low compliance rate of the vaccinees and a heavy economic burden for the poor. In April 2022, India launched the world's first novel rabies vaccine, which is also the only recombinant protein vaccine. Its immunization program involves three injections after the exposure, further shortening the immunization program and reducing the immunization dosage.

At present, there is no rabies recombinant protein vaccine approved in China, so it is of great significance to develop a rabies vaccine with higher efficacy, higher safety, and long-lasting immunity.

### SUMMARY

In view of the technical problems described above, the rabies virus G protein ectodomain is used as a target protein for engineering according to the present disclosure to obtain a G protein antigen with a stable conformation and potent immunogenicity.

### A. Viral membrane fusion protein

The rabies virus G protein is a viral membrane fusion protein. A critical step in enveloped virus infection is the fusion between the viral envelope and the host cell membrane, which is mediated by viral membrane fusion proteins. Viral membrane fusion proteins are classified into type I (e.g., membrane fusion proteins of human immunodeficiency virus and respiratory syncytial virus), type II (e.g., membrane fusion proteins of dengue virus), and type III (e.g., membrane fusion proteins of rhabdovirus and herpesvirus). Viral membrane fusion proteins are conformationally metastable proteins that may transition from a higher-energy pre-fusion conformation to a lower-energy post-fusion conformation via an unstable intermediate state, triggered by certain factors associated with the contact with the host cell. The intermediate state exposes a hydrophobic fusion peptide or fusion loop that may be inserted into the host cell membrane, and the transition to the low-energy post-fusion conformation pulls the envelope and the cell membrane together, promoting the fusion of the viral envelope and the host cell membrane. Since epitopes that stimulate the production of neutralizing antibodies are often contained in the pre-fusion conformation and, in some cases, are absent in the post-fusion form, the immunization with membrane fusion proteins in the pre-fusion conformation is clinically desirable as it has the potential to induce more potent neutralizing antibodies than the immunization with proteins of the post-fusion conformation.

The rabies virus G protein is a type III viral membrane fusion protein. Specifically, the rabies virus G protein (RABV-G for short) has two structural states: a pre-fusion conformation of a bent, hairpin structure on the surface of the virus and a post-fusion conformation that mediates the membrane fusion expansion conformation. RABV-G with the natural structure is in a pre-fusion conformation under a basic condition and a post-fusion conformation under an acidic condition, such that the structure of RABV-G is unstable. Also, the pre-fusion conformation will gradually transition to the post-fusion conformation over time during the storage, leading to a reduced amount of antigenic sites and thus a decrease in immunogenicity. It can be seen that the conformation of the G protein, as the main antigen of rabies virus, can affect the immunogenicity, such that the stabilization of the pre-fusion conformation of the G protein can effectively improve the immunogenicity and enhance the immune protection effect.

### B. Wild-type RABV-G

The present disclosure provides a wild-type RABV-G, comprising a full-length amino acid sequence derived from the G protein (Genbank Accession No. AAA47218.1) of the Pasteur Virus strain (i.e., the PV strain).

The present disclosure provides a wild-type RABV-G ectodomain, comprising an amino acid sequence derived from the amino acids at positions 20-459 of the G protein (Genbank accession No. AAA47218.1) of the PV strain.

In some embodiments, the wild-type RABV-G of the present disclosure is derived from a strain other than the PV strain.

Optionally, the wild-type RABV-G according to the present disclosure is derived from a G protein of the following strains: Pitman Moore (GenBank Accession No. CAI43218 .1), HEP-Flury (GenBank Accession No. BAC53868.1), MOR1-DG (GenBank Accession No. AAK92057.1), MOR3-HM (GenBank Accession No. AAK92058.1), NeiMeng1025C (GenBank Accession No. ABY19509.2), FRA1-FX (GenBank Accession No. AAK92050 .1), CNX8601 (GenBank Accession No. AAG34722 .1), CHI1-BK (GenBank Accession No. AAK92060.1), MAU1-CL (GenBank Accession No. AAK92072 .1), Yunnan_Zt07 (GenBank Accession No. ABX79939.1), RVD (GenBank Accession No. AAO72525.1), nukovo-32 (GenBank Accession No. OP642459), CVS-11 (GenBank Serial No. ADJ29911.1), or CTN (GenBank Accession No. AY009100) strain.

In some embodiments, the present disclosure provides a wild-type RABV-G ectodomain, comprising an amino acid sequence having a sequence corresponding to or aligned with the amino acids at positions 20-459 of the G protein of the PV strain described above.

In some embodiments, the amino acid sequence of the wild-type RABV-G ectodomain of the present disclosure comprises the amino acid sequence set forth in SEQ ID NO: 1. Preferably, the amino acid sequence of wild-type RABV-G ectodomain of the present disclosure is set forth in SEQ ID NO: 1.

The term "corresponding" or "aligned" has the meaning commonly understood by those of ordinary skill in the art. Specifically, "correspond to" or "align with" means that after a reference sequence is aligned with a given sequence by homology or sequence identity, the same residues of the two can be obtained, and the positions of identical residues in the given sequence are the positions corresponding to or aligned with those of the reference sequence. For example, those skilled in the art can easily perform homology alignment on G proteins of other strains and the G protein of the PV strain using common programs such as Align to obtain positions corresponding to or aligned with the amino acids 20-459 of the G protein of the PV strain in the G proteins in other strains.

In some embodiments, the present disclosure provides a wild-type RABV-G ectodomain, comprising an amino acid sequence having at least 90% identity to SEQ ID NO: 1.

Preferably, the present disclosure provides a wild-type RABV-G ectodomain, comprising an amino acid sequence having at least 95%, at least 96%, at least 97%, at least 98%, or at least 99% identity to SEQ ID NO: 1.

### C. RABV-G mutant

The present disclosure provides a RABV-G mutant, comprising a RABV-G ectodomain mutant.

In some embodiments, the RABV-G mutant provided herein has improved stability of the pre-fusion conformation.

Preferably, the RABV-G mutant of the present disclosure is specifically a RABV-G ectodomain mutant having improved stability of the pre-fusion conformation.

As a non-limiting example, the stability of the pre-fusion conformation of the RABV-G mutant can be determined by incubating the RABV-G mutant with a specific antibody of the pre-fusion conformation at a neutral or acidic pH and measuring the antibody binding by ELISA or FACS.

The present disclosure provides a RABV-G ectodomain mutant, wherein the RABV-G ectodomain mutant has one, two, or more amino acid mutations or substitutions at positions 261, 266, 270, 271, 384, or 424 of the wild-type RABV-G ectodomain described above, or has one, two, or more amino acid mutations or substitutions at positions corresponding to or aligned with the amino acid positions described above. The mutation or substitution can improve the stability of the pre-fusion conformation of the G protein.

In some embodiments, the present disclosure provides a RABV-G ectodomain mutant, wherein the RABV-G ectodomain mutant has two amino acid mutations or substitutions at positions 261, 266, 270, 271, 384, or 424 of the wild-type RABV-G ectodomain described above, or has two amino acid mutations or substitutions at positions corresponding to or aligned with the amino acid positions described above. The mutation or substitution can improve the stability of the pre-fusion conformation of the G protein.

Preferably, the two amino acid mutations or substitutions are selected from the following combinations: H261 + H270 or D266 + H270.

In some embodiments, the present disclosure provides a RABV-G ectodomain mutant, wherein the RABV-G ectodomain mutant has one, two, or more amino acid mutations or substitutions at positions 261, 266, 270, 271, 384, or 424 of SEQ ID NO: 1. The mutation or substitution can improve the stability of the pre-fusion conformation of the G protein.

In some embodiments, the present disclosure provides a RABV-G ectodomain mutant, wherein the RABV-G ectodomain mutant has two amino acid mutations or substitutions at positions 261, 266, 270, 271, 384, or 424 of SEQ ID NO: 1. The mutation or substitution can improve the stability of the pre-fusion conformation of the G protein.

Preferably, the two mutations or substitutions of SEQ ID NO: 1 described above are selected from the following combinations: H261 + H270 or D266 + H270.

Further, the residue at the position of the mutation or substitution is mutated into proline or substituted by proline.

In some embodiments, the residue at the position of the mutation or substitution is mutated into leucine or substituted by leucine.

The present disclosure provides a RABV-G ectodomain mutant, wherein the RABV-G ectodomain mutant has one, two, or more amino acid mutations or substitutions selected from H261L, D266P, H270P, L271P, H384P, and H424L in SEQ ID NO: 1. The mutation or substitution can improve the stability of the pre-fusion conformation of the G protein.

In some embodiments, the RABV-G ectodomain mutant has two amino acid mutations or substitutions selected from H261L, D266P, H270P, L271P, H384P, and H424L in SEQ ID NO: 1. The mutation or substitution can improve the stability of the pre-fusion conformation of the G protein.

Preferably, the two mutations or substitutions in SEQ ID NO: 1 described above are selected from the following combinations: H261L + H270P or D266P + H270P.

The present disclosure provides a RABV-G ectodomain mutant, wherein the RABV-G ectodomain mutant comprises the amino acid sequence set forth in any one of SEQ ID NOs: 2-9, as detailed in Table 1.

In some embodiments, the amino acid sequence of the RABV-G ectodomain mutant of the present disclosure is set forth in any one of SEQ ID NOs: 2-9.

Preferably, the amino acid sequence of the RABV-G ectodomain mutant of the present disclosure is set forth in any one of SEQ ID NOs: 2 and 4-9.

Preferably, the amino acid sequence of the RABV-G ectodomain mutant of the present disclosure is set forth in any one of SEQ ID NOs: 4, 5, 8, and 9.

The RABV-G mutant described herein further comprises glycosylation, and the RABV-G mutant can be modified by N-glycosylation at position Asn37, Asn247, or Asn319 of the ectodomain.

In some embodiments, the RABV-G mutant of the present disclosure is modified by N-glycosylation at position Asn319 of the ectodomain, or further modified by N-glycosylation at position Asn37 or Asn247.

In some embodiments, the RABV-G mutant described herein is capable of inducing the production of a neutralizing antibody.

The neutralizing antibody described herein refers to an antibody that can eliminate the virus infectivity after binding to a virus. After the surface antigen of the enveloped virus binds to the neutralizing antibody, the complement can be activated, resulting in the lysis of the virus.

Preferably, the RABV-G mutant described herein is capable of inducing the production of an IgG.

Preferably, the RABV-G mutant described herein is capable of inducing the production of a neutralizing antibody IgG.

Preferably, the RABV-G mutant described herein induces a titer of the produced neutralizing antibody higher than that of a commercially available rabies vaccine.

In some embodiments, the RABV-G mutant described herein can achieve soluble expression and has correct spatial folding.

In some embodiments, a signal peptide is introduced at one terminus of the RABV-G mutant described herein for the extracellular secretory expression of the protein.

Preferably, the signal peptide is introduced into the N-terminus of the RABV-G mutant described herein, and the amino acid sequence of the signal peptide is set forth in MEFGLSWVFLVAIIKGVQC (SEQ ID NO: 18).

In some embodiments, a 6×His tag is introduced at one terminus of the RABV-G mutant described herein for the purification of the recombinantly expressed protein.

Preferably, the 6×His tag is introduced at the C-terminus of the RABV-G mutant described above.

Optionally, the RABV-G mutant described herein is linked to the 6×His tag via a linker. Optionally, the linker described above is selected from the amino acid sequences of (GGGGS)ₙ, (GSGGSG)ₙ, or (GGS)ₙ, wherein n may be an integer greater than 0 and less than or equal to 5. In some embodiments, the linker is preferably GSGGSG.

**Table 1: Amino acid sequences of wild-type and mutant RABV-G ectodomains of the present disclosure**

| Molecule name | Mutation site | Amino acid sequence |
|---|---|---|
| RABV-G1 | Wild-type | KFPIYTIPDKLGPWSPIDIHHLSCPNNLVVEDEGCTNLSGFSYM ELKVGYISAIKMNGFTCTGVVTEAETYTNFVGYVTTTFKRKHF RPTPDACRAAYNWKMAGDPRYEESLHNPYPDYHWLRTVKTT KESLVIISPSVADLDPYDRSLHSRVFPGGNCSGVAVSSTYCSTN HDYTIWMPENPRLGMSCDIFTNSRGKRASKGSETCGFVDERG LYKSLKGACKLKLCGVLGLRLMDGTWVAMQTSNETKWCPP GQLVNLHDFRSDEIEHLVVEELVKKREECLDALESIMTTKSVS FRRLSHLRKLVPGFGKAYTIFNKTLMEADAHYKSVRTWNEIIP SKGCLRVGGRCHPHVNGVFFNGIILGPDGNVLIPEMQSSLLQQ HMELLVSSVIPLMHPLADPSTVFKNGDEAEDFVEVHLPDVHE RISGVDLGLPNWGKY (SEQ ID NO: 1) |
| RABV-G6 | H270P | KFPIYTIPDKLGPWSPIDIHHLSCPNNLVVEDEGCTNLSGFSYM ELKVGYISAIKMNGFTCTGVVTEAETYTNFVGYVTTTFKRKHF RPTPDACRAAYNWKMAGDPRYEESLHNPYPDYHWLRTVKTT KESLVIISPSVADLDPYDRSLHSRVFPGGNCSGVAVSSTYCSTN HDYTIWMPENPRLGMSCDIFTNSRGKRASKGSETCGFVDERG LYKSLKGACKLKLCGVLGLRLMDGTWVAMQTSNETKWCPP GQLVNLHDFRSDEIEPLVVEELVKKREECLDALESIMTTKSVSF RRLSHLRKLVPGFGKAYTIFNKTLMEADAHYKSVRTWNEIIPS KGCLRVGGRCHPHVNGVFFNGIILGPDGNVLIPEMQSSLLQQH MELLVSSVIPLMHPLADPSTVFKNGDEAEDFVEVHLPDVHERI SGVDLGLPNWGKY (SEQ ID NO: 2) |
| RABV-G25 | H261L | KFPIYTIPDKLGPWSPIDIHHLSCPNNLVVEDEGCTNLSGFSYM ELKVGYISAIKMNGFTCTGVVTEAETYTNFVGYVTTTFKRKHF RPTPDACRAAYNWKMAGDPRYEESLHNPYPDYHWLRTVKTT KESLVIISPSVADLDPYDRSLHSRVFPGGNCSGVAVSSTYCSTN HDYTIWMPENPRLGMSCDIFTNSRGKRASKGSETCGFVDERG LYKSLKGACKLKLCGVLGLRLMDGTWVAMQTSNETKWCPP GQLVNLLDFRSDEIEHLVVEELVKKREECLDALESIMTTKSVS FRRLSHLRKLVPGFGKAYTIFNKTLMEADAHYKSVRTWNEIIP SKGCLRVGGRCHPHVNGVFFNGIILGPDGNVLIPEMQSSLLQQ HMELLVSSVIPLMHPLADPSTVFKNGDEAEDFVEVHLPDVHE RISGVDLGLPNWGKY (SEQ ID NO: 3) |
| RABV-G26 | D266P | KFPIYTIPDKLGPWSPIDIHHLSCPNNLVVEDEGCTNLSGFSYM ELKVGYISAIKMNGFTCTGVVTEAETYTNFVGYVTTTFKRKHF RPTPDACRAAYNWKMAGDPRYEESLHNPYPDYHWLRTVKTT KESLVIISPSVADLDPYDRSLHSRVFPGGNCSGVAVSSTYCSTN HDYTIWMPENPRLGMSCDIFTNSRGKRASKGSETCGFVDERG LYKSLKGACKLKLCGVLGLRLMDGTWVAMQTSNETKWCPP GQLVNLHDFRSPEIEHLVVEELVKKREECLDALESIMTTKSVSF RRLSHLRKLVPGFGKAYTIFNKTLMEADAHYKSVRTWNEIIPS KGCLRVGGRCHPHVNGVFFNGIILGPDGNVLIPEMQSSLLQQH MELLVSSVIPLMHPLADPSTVFKNGDEAEDFVEVHLPDVHERI SGVDLGLPNWGKY (SEQ ID NO: 4) |
| RABV-G28 | H384P | KFPIYTIPDKLGPWSPIDIHHLSCPNNLVVEDEGCTNLSGFSYM ELKVGYISAIKMNGFTCTGVVTEAETYTNFVGYVTTTFKRKHF RPTPDACRAAYNWKMAGDPRYEESLHNPYPDYHWLRTVKTT KESLVIISPSVADLDPYDRSLHSRVFPGGNCSGVAVSSTYCSTN HDYTIWMPENPRLGMSCDIFTNSRGKRASKGSETCGFVDERG LYKSLKGACKLKLCGVLGLRLMDGTWVAMQTSNETKWCPP GQLVNLHDFRSDEIEHLVVEELVKKREECLDALESIMTTKSVS FRRLSHLRKLVPGFGKAYTIFNKTLMEADAHYKSVRTWNEIIP SKGCLRVGGRCHPHVNGVFFNGIILGPDGNVLIPEMQSSLLQQ PMELLVSSVIPLMHPLADPSTVFKNGDEAEDFVEVHLPDVHER ISGVDLGLPNWGKY (SEQ ID NO: 5) |
| RABV-G30 | H424L | KFPIYTIPDKLGPWSPIDIHHLSCPNNLVVEDEGCTNLSGFSYM ELKVGYISAIKMNGFTCTGVVTEAETYTNFVGYVTTTFKRKHF RPTPDACRAAYNWKMAGDPRYEESLHNPYPDYHWLRTVKTT KESLVIISPSVADLDPYDRSLHSRVFPGGNCSGVAVSSTYCSTN HDYTIWMPENPRLGMSCDIFTNSRGKRASKGSETCGFVDERG LYKSLKGACKLKLCGVLGLRLMDGTWVAMQTSNETKWCPP GQLVNLHDFRSDEIEHLVVEELVKKREECLDALESIMTTKSVS FRRLSHLRKLVPGFGKAYTIFNKTLMEADAHYKSVRTWNEIIP SKGCLRVGGRCHPHVNGVFFNGIILGPDGNVLIPEMQSSLLQQ HMELLVSSVIPLMHPLADPSTVFKNGDEAEDFVEVHLPDVLER ISGVDLGLPNWGKY (SEQ ID NO: 6) |
| RABV-G32 | H270P+ H261L | KFPIYTIPDKLGPWSPIDIHHLSCPNNLVVEDEGCTNLSGFSYM ELKVGYISAIKMNGFTCTGVVTEAETYTNFVGYVTTTFKRKHF RPTPDACRAAYNWKMAGDPRYEESLHNPYPDYHWLRTVKTT KESLVIISPSVADLDPYDRSLHSRVFPGGNCSGVAVSSTYCSTN HDYTIWMPENPRLGMSCDIFTNSRGKRASKGSETCGFVDERG LYKSLKGACKLKLCGVLGLRLMDGTWVAMQTSNETKWCPP |
| | | GQLVNLLDFRSDEIEPLVVEELVKKREECLDALESIMTTKSVSF RRLSHLRKLVPGFGKAYTIFNKTLMEADAHYKSVRTWNEIIPS KGCLRVGGRCHPHVNGVFFNGIILGPDGNVLIPEMQSSLLQQH MELLVSSVIPLMHPLADPSTVFKNGDEAEDFVEVHLPDVHERI SGVDLGLPNWGKY (SEQ ID NO: 7) |
| RABV-G33 | H270P+ D266P | KFPIYTIPDKLGPWSPIDIHHLSCPNNLVVEDEGCTNLSGFSYM ELKVGYISAIKMNGFTCTGVVTEAETYTNFVGYVTTTFKRKHF RPTPDACRAAYNWKMAGDPRYEESLHNPYPDYHWLRTVKTT KESLVIISPSVADLDPYDRSLHSRVFPGGNCSGVAVSSTYCSTN HDYTIWMPENPRLGMSCDIFTNSRGKRASKGSETCGFVDERG LYKSLKGACKLKLCGVLGLRLMDGTWVAMQTSNETKWCPP GQLVNLHDFRSPEIEPLVVEELVKKREECLDALESIMTTKSVSF RRLSHLRKLVPGFGKAYTIFNKTLMEADAHYKSVRTWNEIIPS KGCLRVGGRCHPHVNGVFFNGIILGPDGNVLIPEMQSSLLQQH MELLVSSVIPLMHPLADPSTVFKNGDEAEDFVEVHLPDVHERI SGVDLGLPNWGKY (SEQ ID NO: 8) |
| RABV-G36 | L271P | KFPIYTIPDKLGPWSPIDIHHLSCPNNLVVEDEGCTNLSGFSYM ELKVGYISAIKMNGFTCTGVVTEAETYTNFVGYVTTTFKRKHF RPTPDACRAAYNWKMAGDPRYEESLHNPYPDYHWLRTVKTT KESLVIISPSVADLDPYDRSLHSRVFPGGNCSGVAVSSTYCSTN HDYTIWMPENPRLGMSCDIFTNSRGKRASKGSETCGFVDERG LYKSLKGACKLKLCGVLGLRLMDGTWVAMQTSNETKWCPP GQLVNLHDFRSDEIEHPVVEELVKKREECLDALESIMTTKSVS FRRLSHLRKLVPGFGKAYTIFNKTLMEADAHYKSVRTWNEIIP SKGCLRVGGRCHPHVNGVFFNGIILGPDGNVLIPEMQSSLLQQ HMELLVSSVIPLMHPLADPSTVFKNGDEAEDFVEVHLPDVHE RISGVDLGLPNWGKY (SEQ ID NO: 9) |

### D. RABV-G trimer and multimer

The present disclosure provides a RABV-G trimer, composed of the RABV-G mutant described herein.

In some embodiments, the RABV-G trimer described herein is composed of the RABV-G ectodomain mutant described herein.

Preferably, the RABV-G trimer of the present disclosure is formed by the self-assembly of the RABV-G mutant of the present disclosure.

The present disclosure provides a method for preparing the RABV-G trimer as follows: recombinantly expressing the RABV-G mutant using a prokaryotic expression system or a eukaryotic expression system, the recombinant RABV-G mutant self-assembling to obtain the RABV-G trimer.

In some embodiments, the present disclosure provides a method for preparing the RABV-G trimer as follows: recombinantly expressing the RABV-G ectodomain mutant using a prokaryotic expression system or a eukaryotic expression system, the recombinant RABV-G ectodomain mutant self-assembling to obtain the RABV-G trimer.

The present disclosure provides a RABV-G multimer, composed of the RABV-G trimer described herein.

In some embodiments, the RABV-G multimer of the present disclosure includes a RABV-G hexamer, nonamer, dodecamer, etc.

The present disclosure provides a method for preparing the RABV-G trimer or multimer, comprising:
(1) recombinantly expressing the RABV-G mutant described herein in a CHO cell by a conventional technique of molecular cloning;
(2) expressing in an expression medium for a certain period of time, harvesting and centrifuging a cell supernatant, and filtering the supernatant through a membrane;
(3) purifying the filtered supernatant on a nickel column;
(4) collecting a sample comprising the target protein, concentrating by ultrafiltration, and subjecting to a first size-exclusion chromatographic (SEC) purification; optionally, purifying a product of the first SEC purification on an anion exchange column, and then subjecting to a second SEC purification;
(5) diluting the purified target protein in a buffer to obtain a soluble trimer or multimer with a uniform particle size.

In some embodiments, in step (4) described above, the buffer used in the first SEC purification is TBS, and the buffer used in the second SEC purification comprises Tween at a certain concentration.

In some embodiments, the buffer in step (5) described above is TBS or a buffer containing Tween.

Further, in steps (4) and (5), the amount of Tween is 0.02%-1%.

Preferably, in steps (4) and (5), the amount of the Tween is 0.02%, 0.05%, or 0.1%, and the Tween is selected from Tween-20 or Tween-80, preferably Tween-80.

### E. Immunogenic complex

The present disclosure provides an immunogenic complex, comprising a protein formed by a covalent binding reaction between a RABV-G antigenic component and a particle protein component.

The RABV-G antigenic component described herein is selected from the RABV-G mutant, the RABV-G trimer, or the RABV-G multimer described herein.

In some embodiments, the immunogenic complex of the present disclosure is a virus-like particle, which is a structure assembled from viral proteins and does not contain any nucleic acid.

The present disclosure provides an immunogenic complex, comprising:
(1) an antigenic component, comprising the RABV-G antigenic component described herein; and
(2) a particle protein component, comprising a nanoparticle protein.

The present disclosure provides an immunogenic complex, comprising:
(1) an antigenic component, comprising the RABV-G antigenic component described herein and binding peptide 1; and
(2) a particle protein component, comprising a nanoparticle protein and a binding peptide 2.

The present disclosure provides an immunogenic complex, comprising:
(1) an antigenic component, comprising the RABV-G antigenic component described herein, the linker peptide 1, and the binding peptide 1; and
(2) a particle protein component, comprising a nanoparticle protein, a linker peptide 2, and a binding peptide 2.

In some embodiments, in any one of the immunogenic complexes provided herein, the antigenic component is formed by fusing the RABV-G antigenic component, at the C-terminus, with the binding peptide 1 via the linker peptide 1. The RABV-G mutant forms a fusion protein with the binding peptide 1, and the fusion protein assembles into the RABV-G trimer or multimer. Alternatively, the RABV-G mutant is fused, at the C-terminus, with the binding peptide 1 via the linker peptide 1, and the fusion protein assembles into the RABV-G trimer or multimer.

In some embodiments, in any one of the immunogenic complexes provided herein, the particle protein component is formed by fusing the nanoparticle protein, at the N-terminus, with the binding peptide 2 via the linker peptide 2.

In some embodiments, the antigenic component and the particle protein component are covalently bound to each other via the binding peptide 1 and the binding peptide 2 to form the immunogenic complex.

In some embodiments, in any one of the immunogenic complexes provided herein, the binding peptide 1 comprises the amino acid sequence set forth in AHIVMVDAYKPTK (SEQ ID NO: 19), which is referred to as "4T" hereinafter.

In some embodiments, in any one of the immunogenic complexes provided herein, the binding peptide 2 comprises the amino acid sequence set forth in DSATHIKFSKRDEDGKELAGATMELRDSSGKTISTWISDGQVKD FYLYPGKYTFVETAAPDGYEVATAITFTVNEQGQVTVNGKATKGDAHI (SEQ ID NO: 20), which is referred to as "4C" hereinafter.

In some embodiments, both the antigenic component and the particle protein component comprise a histidine tag.

In some embodiments, in any one of the immunogenic complexes provided herein, the particle protein may be selected from a nanoparticle protein, and further may be selected from a virus-like particle protein; both the antigenic component and the particle protein component can self-assemble by binding to form a particle structure.

In some embodiments, the nanoparticle used in the present disclosure that can self-assemble includes: an NPM particle, a ferritin particle, a virus-like particle formed by a viral structural protein, an I53-50 particle, and the like. The viral structural protein includes bacteriophage capsid protein AP205 and the like.

In some embodiments, in any one of the immunogenic complexes provided herein, the linker peptide 1 comprises the amino acid sequence of (GGGGS)ₙ, (EAAAK)ₙ, (GSGGSG)ₙ, or (GGS)ₙ, wherein n may be an integer greater than 0 and less than or equal to 5. In some embodiments, in any one of the immunogenic complexes provided herein, the linker peptide 1 is preferably GSGGSG.

In some embodiments, in any one of the immunogenic complexes provided herein, the linker peptide 2 comprises the amino acid sequence of (GGS)ₙ, (GGGGS)ₙ, (EAAAK)ₙ, or (GSGGSG)ₙ, wherein n may be an integer greater than 0 and less than or equal to 10. In some embodiments, in any one of the immunogenic complexes provided herein, the linker peptide 2 is preferably GGSGGSGGS or GGSGGSGGSGGS.

In some embodiments, in any one of the above immunogenic complexes provided herein, the particle protein component is a fusion protein formed by fusing the nanoparticle protein, at the N-terminus, with the binding peptide 2 via the linker peptide 2; preferably, the nanoparticle protein is NPM, AP205 capsid protein 3 (AP205), or ferritin protein. Specifically, in some optional embodiments, the binding peptide 2 (i.e., "4C") is linked to the coding gene of the nanoparticle protein via the linker peptide 2, and the construct is inserted into a prokaryotic expression vector (such as pET-28a(+) or pET-30a(+)) for expression in *Escherichia coli* cells, so as to obtain a fusion protein of the binding peptide 2 and the nanoparticle protein. The fusion protein may be purified by chromatography (e.g., anion exchange chromatography and hydrophobic chromatography) to obtain a product. The nanoparticle protein is preferably NPM, AP205, or ferritin.

Preferably, in the immunogenic complex provided herein, the amino acid sequence of the antigenic component is set forth in any one of SEQ ID NOs: 2-9, and the amino acid sequence of the particle protein component is set forth in SEQ ID NO: 22.

Specifically, in some optional embodiments, any one of the above antigenic components is subjected to a covalent binding reaction with the particle protein component under suitable reaction conditions (e.g., at room temperature), and the coupling is achieved by the covalent bonding of the binding peptide 1 of the antigenic component and the binding peptide 2 of the particle protein component, thereby forming the immunogenic complex.

In some embodiments, the present disclosure provides a method for a covalent binding reaction of an antigenic component and a particle protein component, wherein the antigenic component and the particle protein component are mixed in a protein concentration ratio of 6:1 as determined by the BCA method, a 50% sucrose stock solution is added at a final sucrose concentration of about 25%, and a 1 M Tris-HCl stock solution (pH 7.4) is added at 10% of the total reaction volume to stabilize the pH. The binding reaction is carried out at 22 °C for 24 h. The binding product is purified by SEC.

In some embodiments, the present disclosure provides an immunogenic complex, comprising:
(1) an antigenic component, comprising the RABV-G antigenic component described herein, the linker peptide 1, and the binding peptide 1; and
(2) a particle protein component, comprising a nanoparticle protein, a linker peptide 2, and a binding peptide 2.

The linker peptide 1 is any linker peptide (e.g., a flexible linker peptide or a rigid linker peptide) commonly used in the art, including but not limited to, the amino acid sequences of (GGS)ₙ, (GSGGSG)ₙ, (GGGGS)ₙ, and (EAAAK)ₙ. The linker peptide 1 is preferably GSGGSG.

The linker peptide 2 is any linker peptide commonly used in the art (e.g., a flexible linker peptide or a rigid linker peptide), including, but not limited to, the amino acid sequences of (GGS)ₙ, (GGGGS)ₙ, (EAAAK)ₙ, and (GSGGSG)ₙ, wherein n may be an integer greater than 0 and less than or equal to 10, preferably, GGSGGSGGS or GGSGGSGGSGGS; the nanoparticle protein is NPM, AP205, or ferritin.

In some embodiments, in any one of the immunogenic complexes provided herein, the nanoparticle protein NPM comprises the amino acid sequence set forth in SEQ ID NO: 21.

Preferably, in any one of the immunogenic complexes provided herein, the particle protein component comprises NPM-4C, as set forth in SEQ ID NO: 22, which comprises a fusion protein obtained by linking the binding peptide 2 to the nanoparticle protein NPM set forth in SEQ ID NO: 21 via the linker peptide 2.

Further, the present disclosure provides a method for preparing an immunogenic complex, comprising:
(1) separately ligating coding genes of a RABV-G antigenic component and a particle protein component into expression vectors to construct recombinant expression plasmids;
(2) constructing a recombinant host cell capable of expressing the RABV-G antigenic component and the particle protein component in the host cell;
(3) expressing fusion proteins using the recombinant host cell and purifying the recombinant fusion proteins; and
(4) subjecting the antigenic component and the particle protein component described above to a covalent binding reaction, so as to obtain the immunogenic complex.

Preferably, the immunogenic complex obtained in step (4) above is purified to obtain a vaccine drug substance.

Preferably, in step (1) of the method for preparing the immunogenic complex, the plasmid expressing the RABV-G antigenic component may be selected from pcDNA3.4, and the vector expressing the nanoparticle may be selected from pET-28a(+) or pET-30a(+).

In some embodiments, the immunogenic complex provided herein can induce the production of a neutralizing antibody.

In some embodiments, the immunogenic complex provided herein is used for preparing a neutralizing antibody.

The neutralizing antibody described above refers to an antibody that can eliminate the virus infectivity after binding to a virus, preferably IgG. After the surface antigen of the enveloped virus binds to the neutralizing antibody, the complement can be activated, resulting in the lysis of the virus.

In step (2) of the method for preparing the immunogenic complex described herein, the host cell expressing the RABV-G antigenic component is CHO, and the host cell expressing the particle protein component vector is *E. coli*.

In step (3) of the method for preparing the immunogenic complex described herein, reference can be made to Patent No. CN114395015B for the method for purifying the particle protein.

### F. Nucleic acid

The present disclosure provides coding nucleotides for the RABV-G mutant and the RABV-G antigenic component described above, and the specific nucleotide sequences are readily available to those skilled in the art by conventional means such as codon tables.

Preferably, the coding nucleotide of the RABV-G mutant is obtained by codon optimization.

Preferably, the coding nucleotide sequence described above is subjected to codon optimization preferred in a CHO cell strain.

The present disclosure provides coding nucleotides for the particle protein components and the nanoparticle proteins described above, and the specific nucleotide sequences are readily available to those skilled in the art by conventional means such as codon tables.

Preferably, the coding nucleotide sequences of the RABV-G mutants provided herein are set forth in SEQ ID NOs: 10-17, as specifically shown in Table 2.

The present disclosure also provides vectors comprising the nucleotide sequences of the present disclosure, including cloning or expression vectors, and host cells transformed with the vectors.

In some embodiments, the vector used in the present disclosure is selected from pcDNA3.4, pET-28a(+), or pET-30a(+).

In some embodiments, the host cell expressing the antigenic component vector is CHO, and the host cell expressing the particle protein vector is *E. coli*.

**Table 2: Nucleotide sequences of RABV-G ectodomain mutants of the present disclosure**

| Molecule name | Nucleotide sequence |
|---|---|
| RABV-G6 | AAG TTCCCTATCT ACACCATCCC AGACAAGCTC GGACCTTGGT CCCCTATCGA CATCCACCAC CTGTCCTGTC CTAACAACCT GGTGGTGGAA GATGAGGGCT GCACCAACCT GTCTGGCTTC TCCTACATGG AGCTGAAAGT GGGCTACATC AGCGCCATCA AGATGAACGG CTTTACCTGT ACCGGCGTGG TGACCGAGGC TGAGACATAC ACCAACTTCG TGGGCTACGT GACAACCACC TTCAAGAGGA AGCACTTCAG ACCTACCCCT GATGCCTGTA GAGCCGCTTA CAACTGGAAG ATGGCTGGCG ACCCTAGATA CGAGGAATCT CTCCACAACC CCTATCCCGA CTACCACTGG CTGAGAACCG TGAAGACCAC CAAAGAGAGC CTCGTTATCA TCTCTCCTTC CGTGGCTGAT CTGGATCCTT ACGACAGATC CCTGCACTCT CGGGTGTTTC CCGGCGGAAA CTGCTCTGGA GTGGCCGTGT CTAGCACCTA CTGCTCCACC AACCACGACT ACACTATCTG GATGCCTGAG AACCCTCGGC TGGGCATGTC CTGCGACATC TTCACCAACT CCAGAGGCAA ACGGGCCTCT AAGGGCTCCG AAACCTGCGG CTTCGTGGAC GAGCGGGGCC TGTACAAGTC TCTGAAGGGC GCCTGCAAGC TGAAGCTGTG TGGCGTGCTG GGACTGAGAC TGATGGACGG CACCTGGGTG GCCATGCAGA CCTCCAACGA GACAAAGTGG TGCCCTCCTG GCCAGCTGGT GAATCTGCAC GACTTCCGGA GCGATGAGAT TGAGCCTCTC GTCGTGGAAG AGCTGGTCAA GAAGAGAGAG GAATGCCTGG ACGCTCTGGA ATCCATCATG ACCACAAAAT CCGTGTCCTT CCGCAGACTG AGCCACCTGC GGAAGCTGGT GCCTGGCTTC GGCAAGGCCT ACACAATCTT CAACAAGACA TTGATGGAAG CCGACGCCCA CTACAAGAGC GTGCGGACCT GGAACGAAAT CATCCCCTCC AAAGGCTGTC TGAGAGTCGG CGGCAGATGC CACCCTCACG TGAACGGCGT GTTCTTCAAT GGCATCATCC TGGGCCCTGA TGGCAACGTG CTGATCCCAG AGATGCAATC CTCCCTGCTG CAGCAGCACA TGGAACTGCT GGTGTCGTCT |
| | GTGATCCCTC TGATGCACCC CCTGGCCGAC CCCTCTACCG TGTTCAAGAA CGGCGACGAG GCCGAGGACT TTGTCGAAGT GCATCTGCCT GACGTGCACG AGCGGATCTC CGGGGTGGAC CTGGGCCTGC CCAATTGGGG CAAGTAC (SEQ ID NO:10) |
| RABV-G25 | AAGTTCCCCATCTACACCATCCCCGACAAGCTGGGCCCTTGGTCTCCTATCGACA TCCACCATTTGTCCTGTCCTAACAACCTGGTCGTGGAGGACGAGGGCTGCACCAA TCTGTCCGGTTTCTCCTACATGGAACTCAAAGTGGGCTATATCAGCGCCATCAAG ATGAACGGCTTCACCTGTACCGGCGTCGTGACCGAGGCCGAGACCTACACCAAC TTCGTGGGCTACGTGACGACAACCTTCAAGCGGAAGCACTTCCGGCCCACCCCTG ATGCTTGCAGAGCCGCTTACAACTGGAAGATGGCCGGCGATCCTAGATACGAGG AGTCCCTGCACAACCCCTATCCTGACTACCACTGGCTGAGAACCGTGAAGACCAC CAAAGAGTCTCTCGTGATCATCTCTCCTTCTGTGGCTGATCTGGACCCTTACGAC AGAAGCCTGCACTCCAGGGTGTTCCCTGGAGGCAACTGCTCTGGCGTCGCTGTGT CCTCTACCTACTGCTCCACCAACCACGACTACACAATCTGGATGCCAGAGAACCC TCGGCTGGGCATGTCCTGCGACATCTTCACAAACTCCAGAGGCAAAAGAGCTTCT AAAGGCTCCGAAACCTGCGGCTTTGTGGACGAGCGGGGCCTGTACAAGTCTCTG AAGGGCGCCTGCAAGCTGAAGCTGTGTGGCGTGCTGGGACTGAGACTGATGGAC GGCACCTGGGTAGCCATGCAAACTTCCAACGAGACCAAGTGGTGCCCTCCTGGC CAGCTGGTGAACCTGctgGATTTCCGCTCCGACGAAATCGAGCACCTGGTGGTGGA AGAGCTGGTGAAAAAGAGAGAAGAGTGCCTGGACGCTCTGGAATCCATCATGAC CACAAAGTCCGTGTCCTTCCGGCGGCTGTCCCACCTGCGGAAGCTGGTCCCTGGC TTCGGCAAGGCCTACACCATCTTTAACAAGACCCTGATGGAAGCCGATGCCCACT ACAAGTCCGTGCGGACCTGGAACGAGATCATCCCTAGCAAGGGCTGTCTGAGAG TGGGCGGCAGATGCCACCCCCACGTGAATGGCGTGTTCTTCAACGGCATCATCCT GGGACCTGATGGCAACGTGCTGATCCCAGAGATGCAGTCTTCTCTGCTGCAGCAG CACATGGAACTGCTGGTCTCTAGTGTGATTCCTCTGATGCACCCTCTGGCCGACC CCAGCACCGTGTTTAAGAACGGCGACGAGGCTGAGGACTTCGTGGAAGTGCACT TGCCAGATGTTCACGAGAGAATCAGCGGAGTGGACCTGGGCCTGCCTAATTGGG GCAAGTAC (SEQ ID NO:11) |
| RABV-G26 | AAGTTCCCCATCTACACCATCCCCGACAAGCTGGGCCCTTGGTCTCCTATCGACA TCCACCATTTGTCCTGTCCTAACAACCTGGTCGTGGAGGACGAGGGCTGCACCAA TCTGTCCGGTTTCTCCTACATGGAACTCAAAGTGGGCTATATCAGCGCCATCAAG ATGAACGGCTTCACCTGTACCGGCGTCGTGACCGAGGCCGAGACCTACACCAAC TTCGTGGGCTACGTGACGACAACCTTCAAGCGGAAGCACTTCCGGCCCACCCCTG ATGCTTGCAGAGCCGCTTACAACTGGAAGATGGCCGGCGATCCTAGATACGAGG AGTCCCTGCACAACCCCTATCCTGACTACCACTGGCTGAGAACCGTGAAGACCAC CAAAGAGTCTCTCGTGATCATCTCTCCTTCTGTGGCTGATCTGGACCCTTACGAC AGAAGCCTGCACTCCAGGGTGTTCCCTGGAGGCAACTGCTCTGGCGTCGCTGTGT CCTCTACCTACTGCTCCACCAACCACGACTACACAATCTGGATGCCAGAGAACCC TCGGCTGGGCATGTCCTGCGACATCTTCACAAACTCCAGAGGCAAAAGAGCTTCT AAAGGCTCCGAAACCTGCGGCTTTGTGGACGAGCGGGGCCTGTACAAGTCTCTG |
| | AAGGGCGCCTGCAAGCTGAAGCTGTGTGGCGTGCTGGGACTGAGACTGATGGAC GGCACCTGGGTAGCCATGCAAACTTCCAACGAGACCAAGTGGTGCCCTCCTGGC CAGCTGGTGAACCTGCATGATTTCCGCTCCcccGAAATCGAGCACCTGGTGGTGGA AGAGCTGGTGAAAAAGAGAGAAGAGTGCCTGGACGCTCTGGAATCCATCATGAC CACAAAGTCCGTGTCCTTCCGGCGGCTGTCCCACCTGCGGAAGCTGGTCCCTGGC TTCGGCAAGGCCTACACCATCTTTAACAAGACCCTGATGGAAGCCGATGCCCACT ACAAGTCCGTGCGGACCTGGAACGAGATCATCCCTAGCAAGGGCTGTCTGAGAG TGGGCGGCAGATGCCACCCCCACGTGAATGGCGTGTTCTTCAACGGCATCATCCT GGGACCTGATGGCAACGTGCTGATCCCAGAGATGCAGTCTTCTCTGCTGCAGCAG CACATGGAACTGCTGGTCTCTAGTGTGATTCCTCTGATGCACCCTCTGGCCGACC CCAGCACCGTGTTTAAGAACGGCGACGAGGCTGAGGACTTCGTGGAAGTGCACT TGCCAGATGTTCACGAGAGAATCAGCGGAGTGGACCTGGGCCTGCCTAATTGGG GCAAGTAC (SEQ ID NO:12) |
| RABV-G28 | AAGTTCCCCATCTACACCATCCCCGACAAGCTGGGCCCTTGGTCTCCTATCGACA TCCACCATTTGTCCTGTCCTAACAACCTGGTCGTGGAGGACGAGGGCTGCACCAA TCTGTCCGGTTTCTCCTACATGGAACTCAAAGTGGGCTATATCAGCGCCATCAAG ATGAACGGCTTCACCTGTACCGGCGTCGTGACCGAGGCCGAGACCTACACCAAC TTCGTGGGCTACGTGACGACAACCTTCAAGCGGAAGCACTTCCGGCCCACCCCTG ATGCTTGCAGAGCCGCTTACAACTGGAAGATGGCCGGCGATCCTAGATACGAGG AGTCCCTGCACAACCCCTATCCTGACTACCACTGGCTGAGAACCGTGAAGACCAC CAAAGAGTCTCTCGTGATCATCTCTCCTTCTGTGGCTGATCTGGACCCTTACGAC AGAAGCCTGCACTCCAGGGTGTTCCCTGGAGGCAACTGCTCTGGCGTCGCTGTGT CCTCTACCTACTGCTCCACCAACCACGACTACACAATCTGGATGCCAGAGAACCC TCGGCTGGGCATGTCCTGCGACATCTTCACAAACTCCAGAGGCAAAAGAGCTTCT AAAGGCTCCGAAACCTGCGGCTTTGTGGACGAGCGGGGCCTGTACAAGTCTCTG AAGGGCGCCTGCAAGCTGAAGCTGTGTGGCGTGCTGGGACTGAGACTGATGGAC GGCACCTGGGTAGCCATGCAAACTTCCAACGAGACCAAGTGGTGCCCTCCTGGC CAGCTGGTGAACCTGCATGATTTCCGCTCCGACGAAATCGAGCACCTGGTGGTGG AAGAGCTGGTGAAAAAGAGAGAAGAGTGCCTGGACGCTCTGGAATCCATCATGA CCACAAAGTCCGTGTCCTTCCGGCGGCTGTCCCACCTGCGGAAGCTGGTCCCTGG CTTCGGCAAGGCCTACACCATCTTTAACAAGACCCTGATGGAAGCCGATGCCCAC TACAAGTCCGTGCGGACCTGGAACGAGATCATCCCTAGCAAGGGCTGTCTGAGA GTGGGCGGCAGATGCCACCCCCACGTGAATGGCGTGTTCTTCAACGGCATCATCC TGGGACCTGATGGCAACGTGCTGATCCCAGAGATGCAGTCTTCTCTGCTGCAGCA GcccATGGAACTGCTGGTCTCTAGTGTGATTCCTCTGATGCACCCTCTGGCCGACC CCAGCACCGTGTTTAAGAACGGCGACGAGGCTGAGGACTTCGTGGAAGTGCACT TGCCAGATGTTCACGAGAGAATCAGCGGAGTGGACCTGGGCCTGCCTAATTGGG GCAAGTAC (SEQ ID NO:13) |
| RABV-G30 | AAGTTCCCCATCTACACCATCCCCGACAAGCTGGGCCCTTGGTCTCCTATCGACA TCCACCATTTGTCCTGTCCTAACAACCTGGTCGTGGAGGACGAGGGCTGCACCAA TCTGTCCGGTTTCTCCTACATGGAACTCAAAGTGGGCTATATCAGCGCCATCAAG ATGAACGGCTTCACCTGTACCGGCGTCGTGACCGAGGCCGAGACCTACACCAAC TTCGTGGGCTACGTGACGACAACCTTCAAGCGGAAGCACTTCCGGCCCACCCCTG |
| | ATGCTTGCAGAGCCGCTTACAACTGGAAGATGGCCGGCGATCCTAGATACGAGG AGTCCCTGCACAACCCCTATCCTGACTACCACTGGCTGAGAACCGTGAAGACCAC CAAAGAGTCTCTCGTGATCATCTCTCCTTCTGTGGCTGATCTGGACCCTTACGAC AGAAGCCTGCACTCCAGGGTGTTCCCTGGAGGCAACTGCTCTGGCGTCGCTGTGT CCTCTACCTACTGCTCCACCAACCACGACTACACAATCTGGATGCCAGAGAACCC TCGGCTGGGCATGTCCTGCGACATCTTCACAAACTCCAGAGGCAAAAGAGCTTCT AAAGGCTCCGAAACCTGCGGCTTTGTGGACGAGCGGGGCCTGTACAAGTCTCTG AAGGGCGCCTGCAAGCTGAAGCTGTGTGGCGTGCTGGGACTGAGACTGATGGAC GGCACCTGGGTAGCCATGCAAACTTCCAACGAGACCAAGTGGTGCCCTCCTGGC CAGCTGGTGAACCTGCATGATTTCCGCTCCGACGAAATCGAGCACCTGGTGGTGG AAGAGCTGGTGAAAAAGAGAGAAGAGTGCCTGGACGCTCTGGAATCCATCATGA CCACAAAGTCCGTGTCCTTCCGGCGGCTGTCCCACCTGCGGAAGCTGGTCCCTGG CTTCGGCAAGGCCTACACCATCTTTAACAAGACCCTGATGGAAGCCGATGCCCAC TACAAGTCCGTGCGGACCTGGAACGAGATCATCCCTAGCAAGGGCTGTCTGAGA GTGGGCGGCAGATGCCACCCCCACGTGAATGGCGTGTTCTTCAACGGCATCATCC TGGGACCTGATGGCAACGTGCTGATCCCAGAGATGCAGTCTTCTCTGCTGCAGCA GCACATGGAACTGCTGGTCTCTAGTGTGATTCCTCTGATGCACCCTCTGGCCGAC CCCAGCACCGTGTTTAAGAACGGCGACGAGGCTGAGGACTTCGTGGAAGTGCAC TTGCCAGATGTTctgGAGAGAATCAGCGGAGTGGACCTGGGCCTGCCTAATTGGG GCAAGTAC (SEQ ID NO:14) |
| RABV-G32 | AAGTTCCCTATCTACACCATCCCAGACAAGCTCGGACCTTGGTCCCCTATCGACA TCCACCACCTGTCCTGTCCTAACAACCTGGTGGTGGAAGATGAGGGCTGCACCAA CCTGTCTGGCTTCTCCTACATGGAGCTGAAAGTGGGCTACATCAGCGCCATCAAG ATGAACGGCTTTACCTGTACCGGCGTGGTGACCGAGGCTGAGACATACACCAAC TTCGTGGGCTACGTGACAACCACCTTCAAGAGGAAGCACTTCAGACCTACCCCTG ATGCCTGTAGAGCCGCTTACAACTGGAAGATGGCTGGCGACCCTAGATACGAGG AATCTCTCCACAACCCCTATCCCGACTACCACTGGCTGAGAACCGTGAAGACCAC CAAAGAGAGCCTCGTTATCATCTCTCCTTCCGTGGCTGATCTGGATCCTTACGAC AGATCCCTGCACTCTCGGGTGTTTCCCGGCGGAAACTGCTCTGGAGTGGCCGTGT CTAGCACCTACTGCTCCACCAACCACGACTACACTATCTGGATGCCTGAGAACCC TCGGCTGGGCATGTCCTGCGACATCTTCACCAACTCCAGAGGCAAACGGGCCTCT AAGGGCTCCGAAACCTGCGGCTTCGTGGACGAGCGGGGCCTGTACAAGTCTCTG AAGGGCGCCTGCAAGCTGAAGCTGTGTGGCGTGCTGGGACTGAGACTGATGGAC GGCACCTGGGTGGCCATGCAGACCTCCAACGAGACAAAGTGGTGCCCTCCTGGC CAGCTGGTGAATCTGCTGGACTTCCGGAGCGATGAGATTGAGCCTCTCGTCGTGG AAGAGCTGGTCAAGAAGAGAGAGGAATGCCTGGACGCTCTGGAATCCATCATGA CCACAAAATCCGTGTCCTTCCGCAGACTGAGCCACCTGCGGAAGCTGGTGCCTGG CTTCGGCAAGGCCTACACAATCTTCAACAAGACATTGATGGAAGCCGACGCCCA CTACAAGAGCGTGCGGACCTGGAACGAAATCATCCCCTCCAAAGGCTGTCTGAG AGTCGGCGGCAGATGCCACCCTCACGTGAACGGCGTGTTCTTCAATGGCATCATC CTGGGCCCTGATGGCAACGTGCTGATCCCAGAGATGCAATCCTCCCTGCTGCAGC AGCACATGGAACTGCTGGTGTCGTCTGTGATCCCTCTGATGCACCCCCTGGCCGA CCCCTCTACCGTGTTCAAGAACGGCGACGAGGCCGAGGACTTTGTCGAAGTGCA |
| | TCTGCCTGACGTGCACGAGCGGATCTCCGGGGTGGACCTGGGCCTGCCCAATTGG GGCAAGTAC (SEQ ID NO:15) |
| RABV-G33 | AAGTTCCCTATCTACACCATCCCAGACAAGCTCGGACCTTGGTCCCCTATCGACA TCCACCACCTGTCCTGTCCTAACAACCTGGTGGTGGAAGATGAGGGCTGCACCAA CCTGTCTGGCTTCTCCTACATGGAGCTGAAAGTGGGCTACATCAGCGCCATCAAG ATGAACGGCTTTACCTGTACCGGCGTGGTGACCGAGGCTGAGACATACACCAAC TTCGTGGGCTACGTGACAACCACCTTCAAGAGGAAGCACTTCAGACCTACCCCTG ATGCCTGTAGAGCCGCTTACAACTGGAAGATGGCTGGCGACCCTAGATACGAGG AATCTCTCCACAACCCCTATCCCGACTACCACTGGCTGAGAACCGTGAAGACCAC CAAAGAGAGCCTCGTTATCATCTCTCCTTCCGTGGCTGATCTGGATCCTTACGAC AGATCCCTGCACTCTCGGGTGTTTCCCGGCGGAAACTGCTCTGGAGTGGCCGTGT CTAGCACCTACTGCTCCACCAACCACGACTACACTATCTGGATGCCTGAGAACCC TCGGCTGGGCATGTCCTGCGACATCTTCACCAACTCCAGAGGCAAACGGGCCTCT AAGGGCTCCGAAACCTGCGGCTTCGTGGACGAGCGGGGCCTGTACAAGTCTCTG AAGGGCGCCTGCAAGCTGAAGCTGTGTGGCGTGCTGGGACTGAGACTGATGGAC GGCACCTGGGTGGCCATGCAGACCTCCAACGAGACAAAGTGGTGCCCTCCTGGC CAGCTGGTGAATCTGCACGACTTCCGGAGCCCTGAGATTGAGCCTCTCGTCGTGG AAGAGCTGGTCAAGAAGAGAGAGGAATGCCTGGACGCTCTGGAATCCATCATGA CCACAAAATCCGTGTCCTTCCGCAGACTGAGCCACCTGCGGAAGCTGGTGCCTGG CTTCGGCAAGGCCTACACAATCTTCAACAAGACATTGATGGAAGCCGACGCCCA CTACAAGAGCGTGCGGACCTGGAACGAAATCATCCCCTCCAAAGGCTGTCTGAG AGTCGGCGGCAGATGCCACCCTCACGTGAACGGCGTGTTCTTCAATGGCATCATC CTGGGCCCTGATGGCAACGTGCTGATCCCAGAGATGCAATCCTCCCTGCTGCAGC AGCACATGGAACTGCTGGTGTCGTCTGTGATCCCTCTGATGCACCCCCTGGCCGA CCCCTCTACCGTGTTCAAGAACGGCGACGAGGCCGAGGACTTTGTCGAAGTGCA TCTGCCTGACGTGCACGAGCGGATCTCCGGGGTGGACCTGGGCCTGCCCAATTGG GGCAAGTAC (SEQ ID NO:16) |
| RABV-G36 | AAGTTCCCCATCTACACCATCCCCGACAAGCTGGGCCCTTGGTCTCCTATCGACA TCCACCATTTGTCCTGTCCTAACAACCTGGTCGTGGAGGACGAGGGCTGCACCAA TCTGTCCGGTTTCTCCTACATGGAACTCAAAGTGGGCTATATCAGCGCCATCAAG ATGAACGGCTTCACCTGTACCGGCGTCGTGACCGAGGCCGAGACCTACACCAAC TTCGTGGGCTACGTGACGACAACCTTCAAGCGGAAGCACTTCCGGCCCACCCCTG ATGCTTGCAGAGCCGCTTACAACTGGAAGATGGCCGGCGATCCTAGATACGAGG AGTCCCTGCACAACCCCTATCCTGACTACCACTGGCTGAGAACCGTGAAGACCAC CAAAGAGTCTCTCGTGATCATCTCTCCTTCTGTGGCTGATCTGGACCCTTACGAC AGAAGCCTGCACTCCAGGGTGTTCCCTGGAGGCAACTGCTCTGGCGTCGCTGTGT CCTCTACCTACTGCTCCACCAACCACGACTACACAATCTGGATGCCAGAGAACCC TCGGCTGGGCATGTCCTGCGACATCTTCACAAACTCCAGAGGCAAAAGAGCTTCT AAAGGCTCCGAAACCTGCGGCTTTGTGGACGAGCGGGGCCTGTACAAGTCTCTG AAGGGCGCCTGCAAGCTGAAGCTGTGTGGCGTGCTGGGACTGAGACTGATGGAC GGCACCTGGGTAGCCATGCAAACTTCCAACGAGACCAAGTGGTGCCCTCCTGGC CAGCTGGTGAACCTGCATGATTTCCGCTCCGACGAAATCGAGCACcctGTGGTGGA AGAGCTGGTGAAAAAGAGAGAAGAGTGCCTGGACGCTCTGGAATCCATCATGAC |
| | CACAAAGTCCGTGTCCTTCCGGCGGCTGTCCCACCTGCGGAAGCTGGTCCCTGGC TTCGGCAAGGCCTACACCATCTTTAACAAGACCCTGATGGAAGCCGATGCCCACT ACAAGTCCGTGCGGACCTGGAACGAGATCATCCCTAGCAAGGGCTGTCTGAGAG TGGGCGGCAGATGCCACCCCCACGTGAATGGCGTGTTCTTCAACGGCATCATCCT GGGACCTGATGGCAACGTGCTGATCCCAGAGATGCAGTCTTCTCTGCTGCAGCAG CACATGGAACTGCTGGTCTCTAGTGTGATTCCTCTGATGCACCCTCTGGCCGACC CCAGCACCGTGTTTAAGAACGGCGACGAGGCTGAGGACTTCGTGGAAGTGCACT TGCCAGATGTTCACGAGAGAATCAGCGGAGTGGACCTGGGCCTGCCTAATTGGG GCAAGTAC    (SEQ ID NO:17) |

### G. Immune composition

The present disclosure provides an immune composition, comprising the RABV-G mutant, the RABV-G trimer, the RABV-G multimer, or the immunogenic complex disclosed herein.

Optionally, the immune composition of the present disclosure further comprises a pharmaceutically acceptable carrier.

Preferably, the pharmaceutically acceptable carrier comprises a stabilizer, an excipient, a surfactant, a buffering agent, and a pH regulator; the stabilizer is sucrose or arginine, the excipient is mannitol, the surfactant is Tween-80, the buffering agent is disodium hydrogen phosphate dihydrate or sodium dihydrogen phosphate dihydrate, and the pH regulator is hydrochloric acid.

In some embodiments, the immune composition provided herein is an injection or a lyophilized formulation, preferably a lyophilized formulation.

In some embodiments, the immune composition described herein comprises one or more of the RABV-G mutant, the RABV-G trimer, the RABV-G multimer, or the immunogenic complex described herein.

In some embodiments, the immune composition of the present disclosure can induce the production of a neutralizing antibody.

In some embodiments, the immune composition of the present disclosure is used for preparing a neutralizing antibody.

The neutralizing antibody described above refers to an antibody that can eliminate the virus infectivity after binding to a virus, preferably IgG. After the surface antigen of the enveloped virus binds to the neutralizing antibody, the complement can be activated, resulting in the lysis of the virus.

### H. Vaccine

The present disclosure provides a vaccine, comprising the immune composition described herein and an adjuvant.

In some embodiments, the vaccine provided herein is a human vaccine.

In some embodiments, the vaccine provided herein is a veterinary vaccine.

The adjuvant used in the present disclosure can enhance the immune response through a number of mechanisms, including: lymphocyte recruitment, stimulation of B and/or T cells, and stimulation of macrophages. The adjuvant increases the titer of the induced antibodies and/or the binding affinity of the induced antibodies relative to the use of the immunogen alone. Preferred adjuvants enhance the intrinsic response to an immunogen without causing a conformational change in the immunogen.

The adjuvant used in the present disclosure is selected from: aqueous adjuvants; mineral adjuvants, including aluminum salts, such as Al(OH)₃, aluminum phosphate, calcium phosphate, and the like; oil-based emulsion adjuvants, including oil-in-water emulsions, water-in-oil emulsions, Freund's adjuvants, MF59, white oil-Span adjuvants, and SAF series; microbial adjuvants, including Corynebacterium parvum, Bacillus Calmette-Guerin, muramyl diphthalide and synthetic lipophilic derivatives thereof, bacterial lipopolysaccharides, bacterial toxins, and lipid-soluble waxy D; liposome adjuvants, including non-phospholipid liposomes, such as Novasomes; cytokine adjuvants, including GM-CSF, IL-2, and IL-12; nucleic acid adjuvants, including CpGDNA; Chinese herbal adjuvants, including propolis, saponin, immunostimulating complexes ISCOMs, polysaccharides, glycosides, and compound traditional Chinese medicines; chemical adjuvants, including levamisole, erythromycin cimetidine, or the like.

In some embodiments, the adjuvant contained in the vaccine of the present disclosure is selected from a toll-like receptor agonist, including poly(I:C), GLA-LSQ, flagellin, and CpG1018.

In some embodiments, the adjuvant contained in the vaccine of the present disclosure is selected from a complex adjuvant, including AS adjuvant systems, such as AS01, AS03 and AS04; covaxin; AS37; PIKACA; and BC adjuvant systems, such as BCG-CpG-DNA.

In some embodiments, the polysaccharide adjuvant of the present disclosure is selected from chitosan or inulin; the immunostimulating complex adjuvant is selected from Matrix-M; the lipid adjuvant is selected from a cationic lipid adjuvant such as CAF01, or an anionic lipid adjuvant such as LPS, monophosphoryl lipid A, and liposomal nanoparticle (LNP); the saponin adjuvant is selected from Quil A or QS21.

In some embodiments, the adjuvant of the present disclosure is selected from a nano-adjuvant, such as a silver nanoparticle and a chiral nano-adjuvant.

In some embodiments, the adjuvant of the present disclosure is selected from ISA201, ISA206, IMS1313, or ISA15A.

In some embodiments, the adjuvant of the present disclosure is selected from a trehalose synthetic derivative, a genetically engineered toxoid, or a biodegradable polymeric microsphere. The genetically engineered toxoid adjuvant is selected from cholera toxin, *E. coli* heat-labile toxin, or tetanus toxoid.

The adjuvant of the present disclosure comprises 3%-5% of squalene, 0.4%-1% of Span 85, 0.4%-1% of Tween 80, 10 mM of citrate or 0.1%-0.5% of sodium citrate, and 0.01%-0.05% of citric acid (w/v). The amount of squalene is preferably 3.5% to 4.5%, more preferably 3.5%, 3.6%, 3.7%, 3.8%, 3.9%, 4.1%, 4.2%, 4.3%, 4.4%, or 4.5% (w/v). The squalene-based adjuvant may be the commercial adjuvant MF59 or SWE. MF59 is an oil-in-water emulsion comprising 4.3% of squalene, 0.5% of Tween -80, and 0.5% of Span 85, and SWE is an oil-in-water emulsion similar in composition to MF59.

The squalene-based water/oil adjuvant used in the specific embodiments of the present disclosure comprises the following components, in percentage by mass: 4.2% of squalene, 0.5% of Span 85, 0.5% of Tween-80, 0.264% of citric acid, and 0.016% of sodium citrate.

Preferably, the adjuvant used in the present disclosure is selected from SWE or MF59. Preferably, the adjuvant used in the present disclosure is selected from AS01, AS03, or AS04.

In some embodiments, the vaccine provided herein can induce the production of a neutralizing antibody.

In some embodiments, the vaccine provided herein is used for preparing a neutralizing antibody.

The neutralizing antibody described above refers to an antibody that can eliminate the virus infectivity after binding to a virus, preferably IgG. After the surface antigen of the enveloped virus binds to the neutralizing antibody, the complement can be activated, resulting in the lysis of the virus.

The present disclosure provides a method for preparing the vaccine for preventing or treating rabies, comprising: subjecting the purified antigenic component to a covalent binding reaction with the particle protein component via the binding peptide 1 and the binding peptide 2, and mixing with a proper adjuvant to form an immune composition product. The amount of the immunogenic complex is 0.25-100 µg/dose, preferably 0.5-50 µg/dose, more preferably 0.5 µg/dose, 1 µg/dose, 2 µg/dose, 3 µg/dose, 4 µg/dose, 5 µg/dose, 10 µg/dose, 15 µg/dose, 20 µg/dose, 25 µg/dose, 30 µg/dose, 35 µg/dose, 40 µg/dose, 45 µg/dose, or 50 µg/dose.

In the method for preparing the vaccine for preventing or treating rabies described herein, the water/oil adjuvant is a squalene-based adjuvant, and the saponin adjuvant comprises QS-21. The drug substance of the vaccine is diluted with a buffer (e.g., TBS) according to dose and then mixed with the adjuvant in a 1:1 volume ratio.

The present disclosure further provides a kit, comprising a rabies vaccine, and a device and a container required for administering the vaccine, specifically including: needle/syringe devices, powder containers, and solvent containers.

### I. Medical use

The present disclosure provides medical use of the RABV-G mutant, the RABV-G trimer, the RABV-G multimer, the immunogenic complex, the immune composition, and the vaccine described above, comprising preventing or treating a related disease caused by rabies virus infection.

Preferably, the RABV-G mutant, the RABV-G trimer, the RABV-G multimer, the immunogenic complex, the immune composition, and the vaccine of the present disclosure are used in pre-exposure prophylaxis and post-exposure prophylaxis of rabies virus infection.

The present disclosure provides a pharmaceutical formulation of the RABV-G mutant, the RABV-G trimer, the RABV-G multimer, the immunogenic complex, the immune composition, and the vaccine described above, for use in preventing or treating a related disease caused by rabies virus infection.

The present disclosure provides a vaccination program for the RABV-G mutant, the RABV-G trimer, the RABV-G multimer, the immunogenic complex, the immune composition, and the vaccine described above. The pre-exposure prophylaxis generally comprises two or three visits, two or three intramuscular injection doses, and regular boosters according to the exposure risk. The post-exposure prophylaxis generally comprises three to five intramuscular doses or four intradermal doses.

Generally, the RABV-G mutant, the RABV-G trimer, the RABV-G multimer, the immunogenic complex, the immune composition, and the vaccine provided herein are directly administered to the subject. The direct delivery may be accomplished by parenteral injection, such as subcutaneous, intraperitoneal, intravenous, intramuscular, intradermal injection, or injection to the interstitial space of a tissue. When given prophylactically, e.g., to a resident in or to a visitor to an endemic region for rabies, the dosing schedule may consist of three doses, two doses or one dose. Alternatively or additionally, one dose is administered prophylactically. When administered therapeutically, e.g., after rabies exposure, the dosing schedule may consist of five doses, four doses, three doses, two doses, or one dose. In one preferred embodiment, one or two doses are administered therapeutically.

### J. Beneficial effects

Compared with the prior art, the present disclosure has the following beneficial effects:
(1) The RABV-G ectodomain mutant of the present disclosure achieves soluble expression of the RABV-G ectodomain and can self-assemble into trimers or multimers, thereby solving the technical problem in the art to a great extent.
(2) The RABV-G ectodomain mutant vaccine of the present disclosure has superior cellular immunity and antibody immune response compared with commercially available human rabies vaccines and canine rabies vaccines.
(3) The RABV-G ectodomain mutant vaccine of the present disclosure can induce the production of neutralizing antibodies at a high titer, and is expected to reduce the number of vaccinations, shorten the immunization procedures, and reduce the immunization dose.
(4) The RABV-G ectodomain mutant vaccine of the present disclosure is a recombinant protein vaccine, which involves no complete viruses in the whole production process and the final product, thus possessing high safety, reduced complexity that may be brought to the production process from the source, improved production efficiency, and reduced cost in mass production.
(5) The inventors also found a method for preparing a protein particle of the RABV-G ectodomain mutant with different particle sizes. The method features ease to operate and protein particles with uniform particle sizes, thus laying a foundation for preparing virus-like particles of different types and different particle sizes.

### BRIEF DESCRIPTION OF THE DRAWINGS

To illustrate the technical solutions in specific embodiments of the present disclosure or in the prior art more clearly, the drawings used for illustrating the specific embodiments or examples are briefly described below.
FIG. 1: the SDS-PAGE identification result of RABV-G, wherein A is RABV-G6, B is RABV-G25, C is RABV-G26, D is RABV-G28, E is RABV-G30, F is RABV-G32, G is RABV-G33, and H is RABV-G36;
FIG. 2: A and B show the electron microscopic negative staining results of G6, C shows the particle size and uniformity detection results, and D shows the HPLC detection results;
FIG. 3: the AUC detection results of G6 samples;
FIG. 4: A and B show the electron microscopic negative staining results of RABV-G25;
FIG. 5: A and B show the electron microscopic negative staining results of RABV-G26;
FIG. 6: A and B show the electron microscopic negative staining results of RABV-G28;
FIG. 7: A and B show the electron microscopic negative staining results of RABV-G30;
FIG. 8: A and B show the electron microscopic negative staining results of RABV-G32;
FIG. 9: A and B show the electron microscopic negative staining results of RABV-G33;
FIG. 10: A and B show the electron microscopic negative staining results of RABV-G36;
FIG. 11: A and B show the electron microscopic negative staining results of G6 after the addition of 0.02% Tween-80;
FIG. 12: A and B show the electron microscopic negative staining results of G6 after the addition of 0.05% Tween-80;
FIG. 13: A and B show the electron microscopic negative staining results of G6 after the addition of 0.1% Tween-80;
FIG. 14: the SDS-PAGE identification results of G6-NPM virus-like particle, wherein A is the virus-like particle with the addition of 0.2% Tween-80, B is the virus-like particle with the addition of 0.05% Tween-80, C is the virus-like particle with the addition of 0.02% Tween-80, and D is the virus-like particle with no Tween-80;
FIG. 15: the neutralizing antibody assay results in the immunized serum of mice receiving G1 or G6;
FIG. 16: the neutralizing antibody assay results in the immunized serum of mice receiving two doses of G6 or four doses of control vaccine;
FIG. 17: the neutralizing antibody assay results in the immunized serum of mice receiving the G protein mutant.

### DETAILED DESCRIPTION

The principles and features disclosed herein are described with reference to the following examples, and the examples provided are only intended to explain the present disclosure and are not intended to limit the scope disclosed herein. Before the detailed description disclosed herein is further provided, it will be appreciated that the protection scope disclosed herein is not limited to the specific embodiments described below; it will also be appreciated that the terms used in the examples herein are intended to describe specific embodiments, rather than limit the protection scope disclosed herein. Procedures without specified conditions in the following examples are generally conducted according to conventional conditions or according to conditions recommended by manufacturers. When numerical ranges are given in the examples, it will be appreciated that, unless otherwise specified in the present disclosure, both endpoints of each of the numerical ranges and any numerical value between the two endpoints can be selected. Unless otherwise defined, all technical and scientific terms used herein have the same meaning as commonly understood by those skilled in the art. In addition to the specific methods, equipment, and materials used in the examples, any methods, equipment, and materials similar or equivalent to those described in the examples herein can also be used to implement the present disclosure, based on the understanding of the prior art by those skilled in the art and the disclosure disclosed herein. The experimental materials used in the following examples were purchased from conventional reagent suppliers, unless otherwise specified.

### Example 1: Expression and purification of RABV-G ectodomain mutant

### I. Expression and purification of RABV-G ectodomain mutant

### 1. Materials:

HisTrap excel was purchased from Cytiva, the SEC column (Superose 6 10/300 GL) was purchased from Cytiva, and the anion exchange column (Hitrap Q FF) was purchased from Cytiva.

### 2. Procedures:

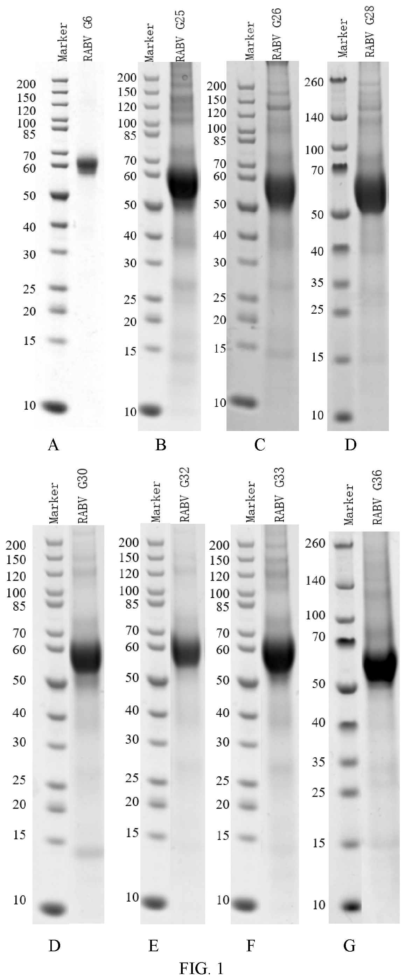

(1) The coding gene of the signal peptide set forth in SEQ ID NO: 18 was introduced at N-termini of the coding genes of RABV-G ectodomain mutants (SEQ ID NOs: 2-9) shown in Table 1, and optionally, the coding gene of the 4T sequence (SEQ ID NO: 19) and the 6×His tag were introduced at C-termini. The fusion gene was obtained by a conventional method of molecular cloning, subjected to codon optimization for CHO cells, and then cloned into the SPB-007 vector to construct a stable transfection cell pool. Positive cell pools were selected and subjected to cell expansion culture. The cell viability was greater than 95%.
(2) After 12 days of expression in the expression medium, the cell supernatant was harvested and collected by centrifugation at 6000 g for 20 min in a centrifuge tube, and the supernatant was filtered through a PES membrane with a pore size of 0.22 µm.
(3) The filtrate supernatant was purified on the Histrap Excel-5 mL nickel column. After loading, the flow-through solution was collected. The column was eluted with 20 mM imidazole and 10 folds (in volume) of TBS buffer, and the sample was collected. Then the column was eluted with 500 mM imidazole and 10 folds (in volume) of TBS buffer, and the eluate sample was collected. The target protein was collected after all samples were identified by SDS-PAGE.
(4) The sample containing the target protein was collected, concentrated in a 10 KD ultrafiltration concentration tube, and further purified using Superose6 increase 10/300 GL SEC column, with TBS as the buffer. The samples were collected and detected by SDS-PAGE electrophoresis, and the results are shown in FIG. 1A. The protein concentration of the sample determined by BCA was maintained at an appropriate temperature for subsequent detection, and the sample was referred to as G6.

The SDS-PAGE identification results of other variants are shown in FIGs. 1B-H, wherein B is RABV-G25, C is RABV-G26, D is RABV-G28, E is RABV-G30, F is RABV-G32, G is RABV-G33, and H is RABV-G36.

(5) Optionally, G6 was further purified. G6 protein purified by SEC was exchanged into a buffer containing 50 mM Tris and 50 mM NaCl, purified on the anion exchange column hitrap Q FF, wherein solution A was a buffer containing 50 mM Tris and 50 mM NaCl, and solution B was a buffer containing 50 mM Tris and 1 M NaCl. Linear gradient elution was performed. The sample was collected and further purified on the Superose6 increase 10/300 GL column, with TBS as the buffer. The target protein was collected and detected by SDS-PAGE electrophoresis, and the protein concentration was determined by the BCA method. The protein was stored at an appropriate temperature for subsequent detection.

### II. Characterization of G6 protein

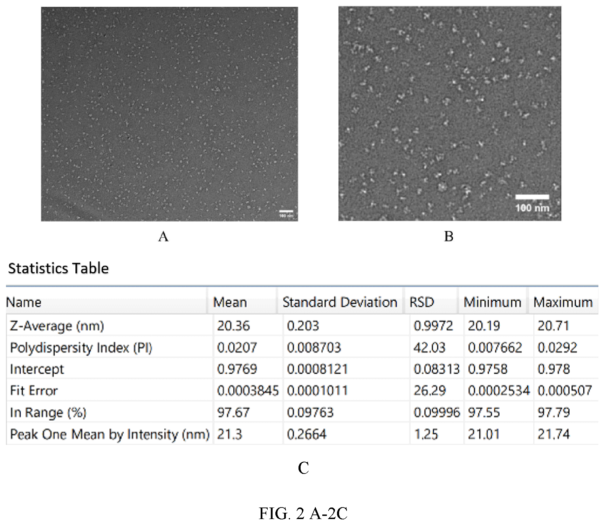

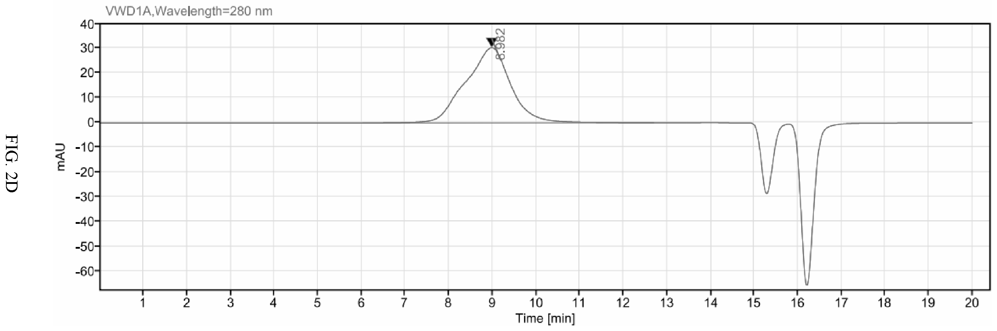

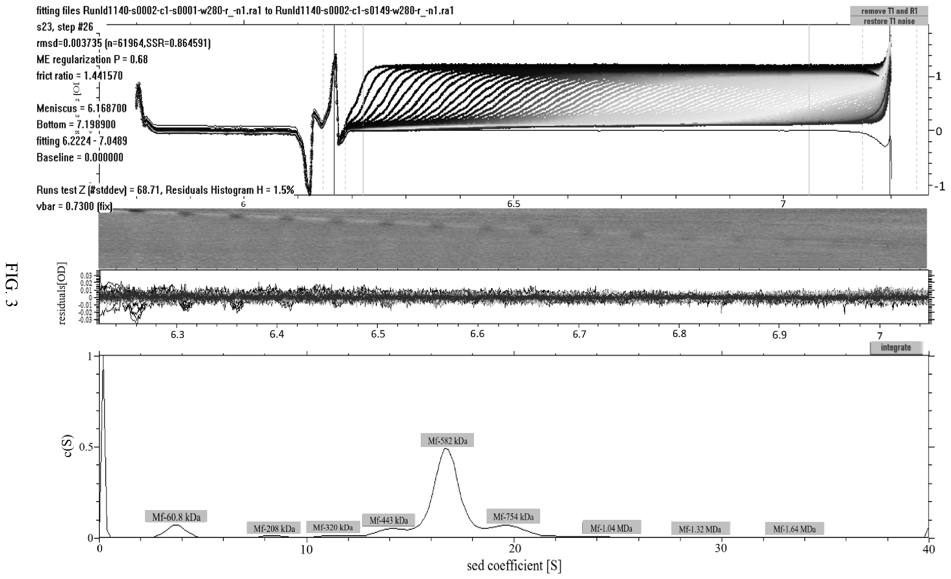

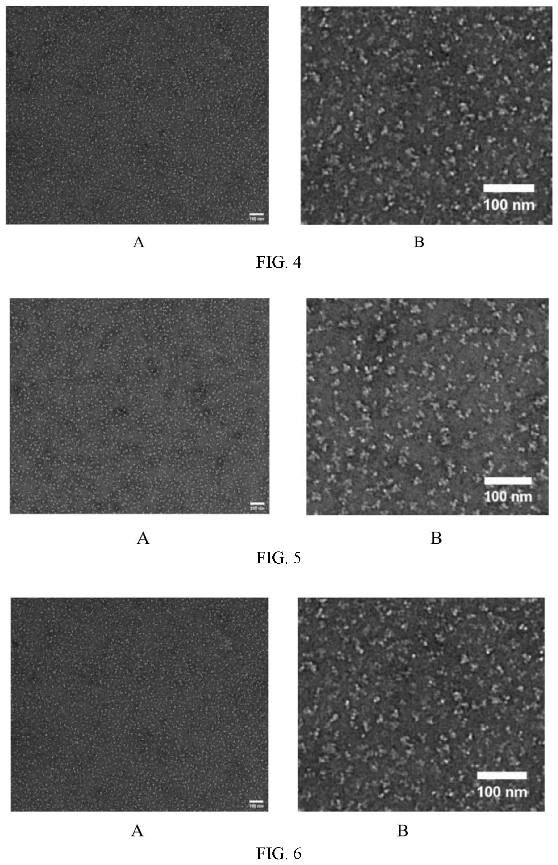

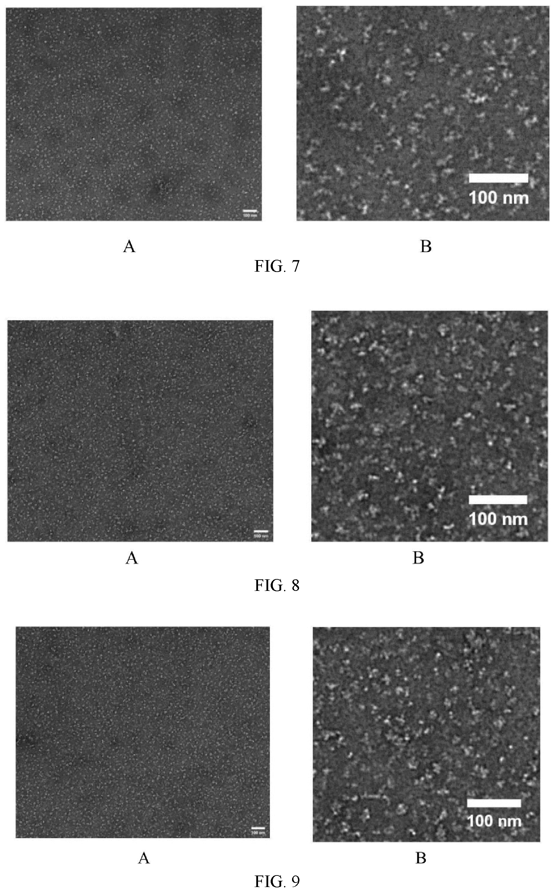

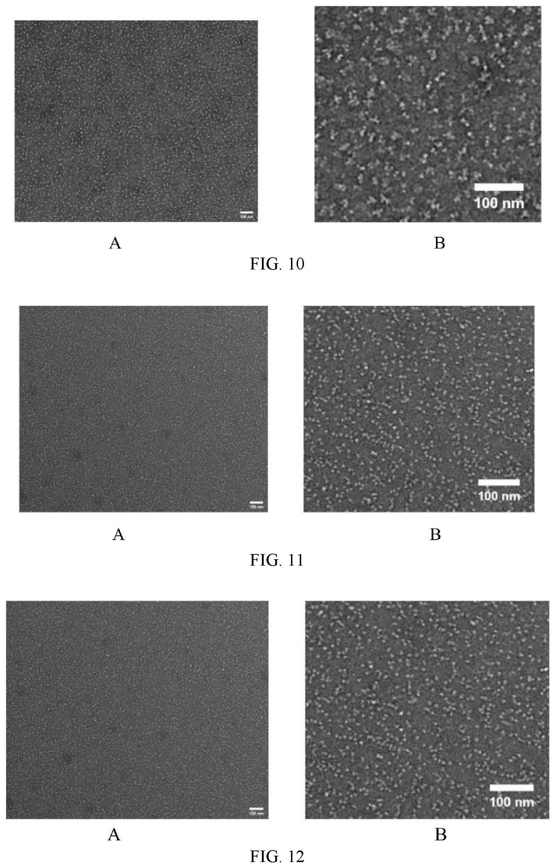

(1) The purified G6 protein was diluted with TBS to 0.01 mg/mL, and negative staining observation was performed. It could be observed that the sample formed relatively uniform nanoparticles, and the results are shown in FIGs. 2A and B. Also, the particle size and uniformity of the particles were detected, and the results are shown in FIG. 2C. The particle exhibited a uniform size of about 20.36 nm. The purity of the G6 protein was detected by HPLC, and the results are shown in FIG. 2D. The main peak pattern of the sample was asymmetric, which may be caused by glycosylation, and the purity was about 89%.
(2) The molecular weight and uniformity of the G6 protein sample were determined by AUC. The results are shown in FIG. 3, wherein the peak with a molecular weight of about 60.8 kDa on the left side was the monomer; 9-mer predominated among the multimers formed by self-assembly with a molecular weight of about 582 kDa, while the 6-mer and the 12-mer accounted for a small portion with molecular weights of about 443 kDa and 754 kDa. It can be seen that G6 can self-assemble into multimers.
(3) Samples of other RABV-G ectodomain mutants purified by nickel and SEC columns were diluted to 0.01 mg/mL and subjected to negative staining observation, and it can be seen that relatively uniform nanoparticles were formed in all the samples. FIGs. 4A and B show the electron microscopic negative staining results of RABV-G25, FIGs. 5A and B show the electron microscopic negative staining results of RABV-G26, FIGs. 6A and B show the electron microscopic negative staining results of RABV-G28, FIGs. 7A and B show the electron microscopic negative staining results of RABV-G30, FIGs. 8A and B show the electron microscopic negative staining results of RABV-G32, FIGs. 9A and B show the electron microscopic negative staining results of RABV-G33, and FIGs. 10A and B show the electron microscopic negative staining results of RABV-G36.

### Example 2: Binding of RABV-G ectodomain mutant to NPM and particle characterization

### I. Preparation of RABV-G ectodomain mutants with different particle sizes

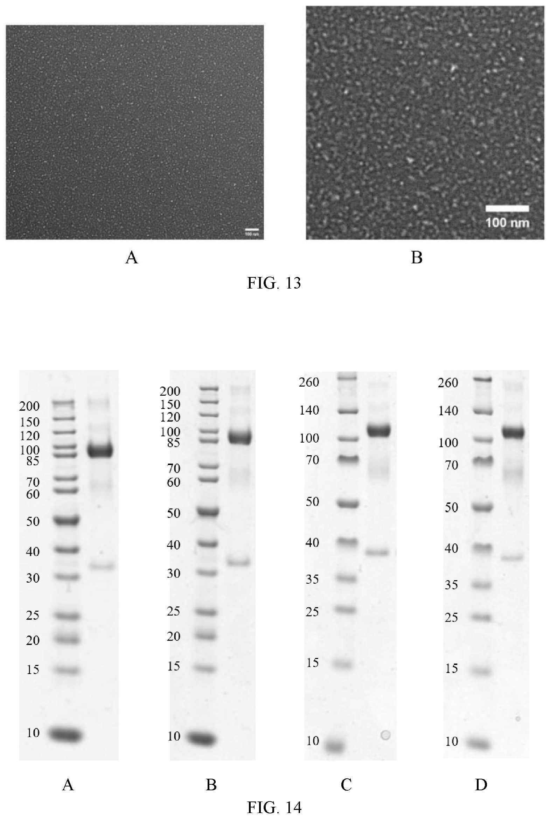

0.02%, 0.05%, or 0.1% Tween-80 was added to the purified G6 protein samples. Negative staining was performed. It can be seen that the dispersed samples formed small protein particles with uniform size. FIGs. 11A and B show the electron microscopic negative staining results of G6 after the addition of 0.02% Tween-80, FIGs. 12A and B show the electron microscopic negative staining results of G6 after the addition of 0.05% Tween-80, and FIGs. 13A and B show the electron microscopic negative staining results of G6 after the addition of 0.1% Tween-80.

### II. Binding assay of RABV-G ectodomain mutant to NPM

The G6 diluted in TBS and the G6 dispersed in Tween at different concentrations obtained in Example 1 were mixed with NPM-4C (SEQ ID NO: 22) as the particle protein component in a BCA protein concentration ratio of 6:1, a 50% sucrose stock solution was added at a final sucrose concentration of about 25%, and a 1 M Tris-HCl stock solution pH 7.4 was added at 10% of the total reaction volume to stabilize the pH. The binding reaction was carried out at 22 °C for 24 h. The bound product was separated and purified on a SEC column (the purification buffer was 20 mM Tris-HCl, 150 mM NaCl, 25% Sucrose, pH 7.4), and the G6-NPM component was collected. The purified G6-NPM virus-like particles were identified by SDS-PAGE, as shown in FIG. 14. A is the virus-like particle formed by dispersing G6 in 0.2% Tween-80, B is the virus-like particle formed by dispersing G6 in 0.05% Tween-80, C is the virus-like particle formed by dispersing G6 in 0.02% Tween-80, and D is the virus-like particle formed by G6 without Tween-80. It can be seen that G6 dispersed in Tween-80 at different concentrations can form virus-like particles with different molecular weights after binding to NPM.

### Example 3: Animal immunization study 1

### (1) Materials

Mice: female BALB/c mice, aged 5-6 weeks (purchased from Guangdong Vital River Laboratory Animal Technology Co., Ltd.)
Adjuvant: SWE;
Pseudovirus: Zhongyan Guobang;
Cell: 293T cell;
Control vaccines: lyophilized human rabies vaccine (Vero cells, Liaoning Chengda), and lyophilized human rabies vaccine (human diploid cells, Chengdu Kanghua);
The other reagents and consumables are all commercially available, conventional reagents and consumables.

### (2) Procedures

As shown in Table 3, the mice were immunized with one vaccine dose by intramuscular injection on days 0 and 7 at 5 µg/dose/mouse, with each vaccine dose containing 25 µg of SWE adjuvant. The serum was isolated from the blood collected on days 7 and 14 for the detection of neutralizing antibodies.

**Table 3: Animal immunization regimen 1**

| Group (n=6) | Antigen | Adjuvant | Dose | Day 0 | Day 7 | Day 7 | Day 14 |
|---|---|---|---|---|---|---|---|
| 1 | RABV-G1 | SWE | 5µg | | | | |
| 2 | RABV-G6 | | | Primary immunization | Interim blood sampling | Secondary immunization | Interim blood sampling |
| 3 | Lyophilized human rabies vaccine (vero cells) | | 1/10 human dose | | | | |
| 4 | Lyophilized human rabies vaccine (human diploid cells) | | | | | | |

### (3) Neutralizing antibody assay:

1. Sample preparation: The mouse serum was inactivated in a dry thermostat at 56 °C for 30 min.

Virus preparation: The pseudovirus was taken from the -80 °C flask, thawed at 4 °C, and diluted to 5 × 10⁴ TCID50/mL in a DMEM complete medium (DMEM + 10% FBS).
2. The serum samples were diluted at a dilution factor of 1:100 in the first well (i.e., 1.5 µL of sample + 148.5 µL of culture medium for dilution, with the final dilution factor in each well being 1:250). Two duplicate wells were set for each serum sample, and the samples were serially 3-fold diluted to a total of 7 concentrations (the last 6 concentrations were used when readings were taken).
3. 50 µL of diluted virus was added to each well at a virus concentration of 5 × 10⁴ TCID50/mL, and blank wells were all blocked with a sterilized liquid at 200 µL/well. The plate was incubated in a cell incubator at 37 °C/5% CO₂ for 1 h;
4. After the samples were co-incubated with virus for 1 h, 293T cells at a density of 0.5 × 10⁶/mL were added at 100 µL/well according to the label, and then the plate was put into a cell incubator at 37 °C/5% CO₂ for 24 h of culturing;
7. Detection: The 96-well white plate and the Bio-Lite detection reagent were taken out and equilibrated to room temperature. 150 µL of the liquid in the plate was discarded by pipetting from each well, and then 100 µL of the detection reagent was added to the plate. The plate was left to stand for 5 min, and the chemiluminescence values (RLU) were detected on a fluorescence microplate reader.

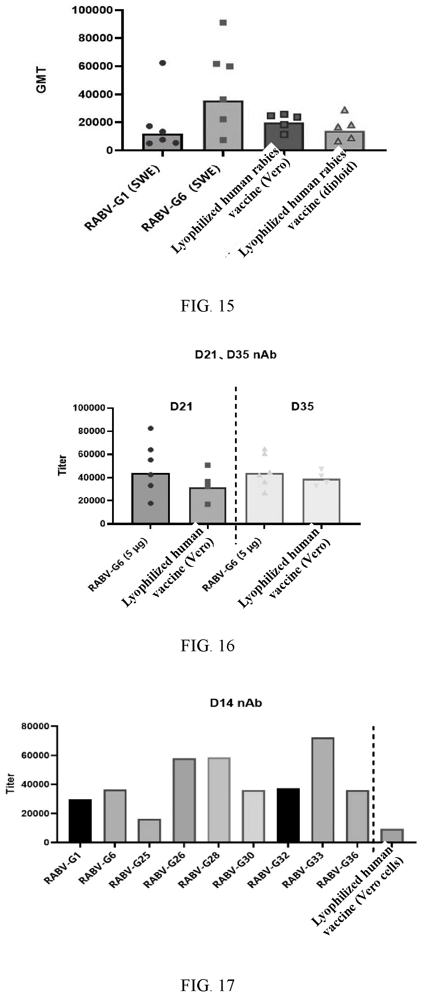

The results are shown in FIG. 15. Comparing G1 (i.e., wild-type) with G6 using the SWE adjuvant, the serum neutralizing antibody titers of mice immunized with G6 on day 14 were significantly higher than those of G1, and were also higher than those of the Vero cell-based lyophilized vaccine and the human diploid-based lyophilized vaccine, indicating that G6 has relatively good immunogenicity.

### Example 4: Animal immunization study 2

### (1) Materials

Mice: female BALB/c mice, aged 5-6 weeks (purchased from Guangdong Vital River Laboratory Animal Technology Co., Ltd.);
Adjuvant A: 4.2% of squalene, 0.5% of Span 85, 0.5% of Tween-80, 0.264% of citric acid, and 0.016% of sodium citrate (w/w);
Pseudovirus: Zhongyan Guobang;
Cell: 293T cell;
Control vaccine: approved lyophilized human rabies vaccine (Vero cells, Liaoning Chengda);
The other reagents and consumables are all commercially available, conventional reagents and consumables.

### (2) Procedures

As shown in Table 4, the mice were immunized with one RABV-G6 vaccine dose by intramuscular injection on days 0 and 7 at 5 µg/dose/mouse, with each vaccine dose containing 25 µg of adjuvant A. The serum was isolated from the blood collected on days 7 and 14 for the detection of neutralizing antibodies. The control vaccine was administered by intramuscular injection on days 0, 7, 14, and 21 at 1/10 human dose/mouse. The serum was separated from the blood samples on days 7, 14, 21, and 35, and neutralizing antibodies were detected.

**Table 4: Animal immunization regimen 2**

| Group (n=6) | Antigen | Adjuvant | Dose | Day 0 | Day 7 | Day 14 | | Day 21 | | Day 35 |
|---|---|---|---|---|---|---|---|---|---|---|
| 1 | RABV-G6 | Adjuvant A | 5 µg | Primary immunization | Interim blood sampling + secondary immunization | Interim blood sampling | NA | Interim blood sampling | NA | Blood sampling at endpoint |
| 7 | Lyophilized human rabies vaccine (vero cells) | NA | 1/10 human dose | Primary immunization | Interim blood sampling + secondary immunization | Interim blood sampling | Tertiary immunization | Interim blood sampling | Quaternary immunization | |

### (3) Neutralizing antibody assay:

1. Sample preparation: The mouse serum was inactivated in a dry thermostat at 56 °C for 30 min.

Virus preparation: The pseudovirus was taken from the -80 °C flask, thawed at 4 °C, and diluted to 5 × 10⁴ TCID50/mL in a DMEM complete medium (DMEM + 10% FBS).
2. The serum samples were diluted at a dilution factor of 1:100 in the first well (i.e., 1.5 µL of sample + 148.5 µL of culture medium for dilution, with the final dilution factor in each well being 1:250). Two duplicate wells were set for each serum sample, and the samples were serially 3-fold diluted to a total of 7 concentrations (the last 6 concentrations were used when readings were taken).
3. 50 µL of diluted virus was added to each well at a virus concentration of 5 × 10⁴ TCID50/mL, and blank wells were all blocked with a sterilized liquid at 200 µL/well. The plate was incubated in a cell incubator at 37 °C/5% CO₂ for 1 h;
4. After the samples were co-incubated with virus for 1 h, 293T cells at a density of 0.5 × 10⁶/mL were added at 100 µL/well according to the label, and then the plate was put into a cell incubator at 37 °C/5% CO₂ for 24 h of culturing;
7. Detection: The 96-well white plate and the Bio-Lite detection reagent were taken out and equilibrated to room temperature. 150 µL of the liquid in the plate was discarded by pipetting from each well, and then 100 µL of the detection reagent was added to the plate. The plate was left to stand for 5 min, and the chemiluminescence values (RLU) were detected on a fluorescence microplate reader.

The results, as shown in FIG. 16, indicate that on days 21 and 35, the neutralizing antibody titers of the RABV-G6 vaccine were higher than those of the control vaccines, suggesting that the immune effect of the RABV-G6 vaccine with two injections was superior to that of the control vaccines with four injections.

### Example 5: Animal immunization study 3

### (1) Materials

Mice: female BALB/c mice, aged 5-6 weeks (purchased from Guangdong Vital River Laboratory Animal Technology Co., Ltd.);
Adjuvant A: 4.2% of squalene, 0.5% of Span 85, 0.5% of Tween-80, 0.264% of citric acid, and 0.016% of sodium citrate (w/w);
Pseudovirus: Zhongyan Guobang;
Cell: 293T cell;
Control vaccine: an approved vero cell-based human rabies vaccine;
The other reagents and consumables are all commercially available, conventional reagents and consumables.

### (2) Procedures

As shown in Table 5, the mice were immunized with one vaccine dose by intramuscular injection on days 0 and 7 at 5 µg/dose/mouse, with each vaccine dose containing 25 µg of adjuvant A. The serum was isolated from the blood collected on days 7 and 14 for the detection of neutralizing antibodies.

**Table 5: Animal immunization regimen 3**

| Group (n=6) | Antigen | Adjuvant | Dose | Day 0 | Day 7 | Day 14 |
|---|---|---|---|---|---|---|
| 1 | RABV-G1 | Adjuvant A | 5µg | | | |
| 2 | RABV-G6 | | | | | |
| 3 | RABV-G25 | | | | | |
| 4 | RABV-G26 | | | | | |
| 5 | RABV-G28 | | | Primary immunization | Interim blood sampling + secondary immunization | Blood sampling at endpoint |
| 6 | RABV-G30 | | | | | |
| 7 | RABV-G32 | | | | | |
| 8 | RABV-G33 | | | | | |
| 9 | RABV-G36 | | | | | |
| 10 | Lyophilized human rabies vaccine (vero cells) | NA | 1/10 human dose | | | |

### (3) Neutralizing antibody assay:

1. Sample preparation: The mouse serum was inactivated in a dry thermostat at 56 °C for 30 min.

Virus preparation: The pseudovirus was taken from the -80 °C flask, thawed at 4 °C, and diluted to 5 × 10⁴ TCID50/mL in a DMEM complete medium (DMEM + 10% FBS).
2. The serum samples were diluted at a dilution factor of 1:100 in the first well (i.e., 1.5 µL of sample + 148.5 µL of culture medium for dilution, with the final dilution factor in each well being 1:250). Two duplicate wells were set for each serum sample, and the samples were serially 3-fold diluted to a total of 7 concentrations (the last 6 concentrations were used when readings were taken).
3. 50 µL of diluted virus was added to each well at a virus concentration of 5 × 10⁴ TCID50/mL, and blank wells were all blocked with a sterilized liquid at 200 µL/well. The plate was incubated in a cell incubator at 37 °C/5% CO₂ for 1 h;
4. After the samples were co-incubated with virus for 1 h, 293T cells at a density of 0.5 × 10⁶/mL were added at 100 µL/well according to the label, and then the plate was put into a cell incubator at 37 °C/5% CO₂ for 24 h of culturing;
7. Detection: The 96-well white plate and the Bio-Lite detection reagent were taken out and equilibrated to room temperature. 150 µL of the liquid in the plate was discarded by pipetting from each well, and then 100 µL of the detection reagent was added to the plate. The plate was left to stand for 5 min, and the chemiluminescence values (RLU) were detected on a fluorescence microplate reader.

The results are shown in FIG. 17. Comparing the serum neutralizing antibody titers of the immunized mice on day 14, the neutralizing antibody titers of all mutants were superior to those of the control vaccines; that is, G6, G26, G28, G30, G32, G33, and G36 were superior to G1 (wild-type).

In summary, the above examples and drawings are only for the purpose of illustrating preferred examples disclosed herein, and are not intended to limit the protection scope of the present disclosure. Any modifications, equivalent substitutions, improvements, and the like made without departing from the spirit and principle of the present disclosure shall all fall within the protection scope of the present disclosure.

## Claims

1. A rabies virus G protein mutant, comprising a mutant of a wild-type rabies virus G protein ectodomain, wherein the mutant of the wild-type rabies virus G protein ectodomain can improve the stability of the pre-fusion conformation of the G protein; preferably, the G protein mutant can induce the generation of a neutralizing antibody.

2. The G protein mutant according to claim 1, wherein the G protein of the wild-type rabies virus is derived from a G protein of the following strains: Pasteur Virus (Genbank Accession No. AAA47218.1), Pitman Moore (GenBank Accession No. CAI43218.1), HEP-Flury (GenBank Accession No. BAC53868.1), MOR1-DG (GenBank Accession No. AAK92057.1), MOR3-HM (GenBank Accession No. AAK92058.1), NeiMeng1025C (GenBank Accession No. ABY19509.2), FRA1-FX (GenBank Accession No. AAK92050.1), CNX8601 (GenBank Accession No. AAG34722.1), CHI1-BK (GenBank Accession No. AAK92060.1), MAU1-CL (GenBank Accession No. AAK92072.1), Yunnan_Zt07 (GenBank Accession No. ABX79939.1), RVD (GenBank Accession No. AAO72525.1), nukovo-32 (GenBank Accession No. OP642459), CVS-11 (GenBank Serial No. ADJ29911.1), or CTN (GenBank Accession No. AY009100) strain; preferably, the wild-type rabies virus G protein is derived from the G protein of Pasteur Virus strain (PV strain).

3. The G protein mutant according to claim 2, wherein the amino acid sequence of the wild-type rabies virus G protein ectodomain is derived from the amino acids at positions 20-459 of the G protein of the PV strain, or comprises a sequence corresponding to or aligned with the amino acids at positions 20-459 of the G protein of the PV strain; preferably, the amino acid sequence of the wild-type rabies virus G protein ectodomain has at least 90%, at least 95%, at least 96%, at least 97%, at least 98%, or at least 99% identity to SEQ ID NO: 1; more preferably, the amino acid sequence of the wild-type rabies virus G protein ectodomain is set forth in SEQ ID NO: 1.

4. The G protein mutant according to any one of claims 1-3, wherein the G protein mutant has one, two, or more amino acid mutations or substitutions at positions 261, 266, 270, 271, 384, or 424 of the wild-type rabies virus G protein ectodomain; preferably, the two amino acid mutations or substitutions are selected from the following combinations: H261 + H270 or D266 + H270.

5. The G protein mutant according to claim 4, wherein the residue at the position of the mutation or substitution is mutated into proline or substituted by proline; alternatively, the residue at the position of the mutation or substitution is mutated into leucine or substituted by leucine.

6. The G protein mutant according to claim 4 or 5, wherein the G protein mutant has one, two, or more amino acid mutations or substitutions of H261L, D266P, H270P, L271P, H384P, and H424L in SEQ ID NO: 1;
preferably, the two mutations or substitutions in SEQ ID NO: 1 are selected from the following combinations: H261L+H270P or D266P+H270P.

7. The G protein mutant according to any one of claims 4-6, wherein the G protein mutant comprises the amino acid sequence set forth in any one of SEQ ID NOs: 2-9; preferably, the amino acid sequence of the G protein mutant is set forth in any one of SEQ ID NOs: 2-9; more preferably, the amino acid sequence of the G protein mutant is set forth in any one of SEQ ID NOs: 2 and 4-9.

8. The G protein mutant according to any one of claims 1-7, wherein a signal peptide or a 6×His tag is introduced at one terminus of the G protein mutant; preferably, the signal peptide is introduced at the N-terminus of the G protein mutant, and/or the 6×His tag is introduced at the C-terminus of the G protein mutant; optionally, the signal peptide comprises the amino acid sequence set forth in SEQ ID NO: 18.

9. A rabies virus G protein trimer or multimer, comprising the rabies virus G protein mutant according to any one of claims 1-8.

10. The G protein trimer or multimer according to claim 9, wherein the trimer or multimer is formed by self-assembly of the rabies virus G protein mutant according to any one of claims 1-8; preferably, the multimer includes a hexamer, a nonamer, and a dodecamer.

11. The G protein trimer or multimer according to claim 9 or 10, wherein the trimer or multimer is prepared by a method comprising the following steps:
(1) recombinantly expressing the rabies virus G protein mutant according to any one of claims 1-8 in a CHO cell by a conventional technique of molecular cloning,
(2) expressing in an expression medium for a certain period of time, harvesting and centrifuging a cell supernatant, and filtering the supernatant through a membrane,
(3) purifying the filtered supernatant on a nickel column,
(4) collecting a sample comprising the target protein, concentrating by ultrafiltration, and subjecting to size-exclusion chromatographic (SEC) purification, and
(5) diluting the purified target protein in a buffer to obtain a soluble G protein trimer or multimer with a uniform particle size;
wherein the buffer used for the SEC purification in step (4) is TBS; optionally, in step (4), the product obtained from a first SEC purification is purified on an anion-exchange column and then subjected to a second SEC purification; the buffer in step (5) is TBS or Tween.

12. The G protein trimer or multimer according to claim 11, wherein step (5) comprises adding a 0.02%-1% Tween to the purified target protein;
optionally, the Tween is selected from Tween-20 or Tween-80;
preferably, the Tween is Tween-80.

13. A method for preparing the G protein trimer or multimer according to any one of claims 9-12, comprising the following steps:
(1) recombinantly expressing the rabies virus G protein mutant according to any one of claims 1-8 in a CHO cell by a conventional technique of molecular cloning,
(2) expressing in an expression medium for a certain period of time, harvesting and centrifuging a cell supernatant, and filtering the supernatant through a membrane,
(3) purifying the filtered supernatant on a nickel column,
(4) collecting a sample comprising the target protein, concentrating by ultrafiltration, and subjecting to SEC purification, and
(5) diluting the purified target protein in a buffer to obtain a soluble G protein trimer or multimer with a uniform particle size;
wherein the buffer used for the SEC purification in step (4) is TBS; optionally, in step (4), the product obtained from a first SEC purification is purified on an anion-exchange column and then subjected to a second SEC purification; the buffer in step (5) is TBS or Tween.

14. The method according to claim 13, wherein step (5) comprises adding a 0.02%-1% Tween to the purified target protein;
optionally, the Tween is selected from Tween-20 or Tween-80;
preferably, the Tween is Tween-80.

15. An immunogenic complex, comprising:
(1) an antigenic component, comprising an antigen selected from the rabies virus G protein mutant according to any one of claims 1-8, or the rabies virus G protein trimer or multimer according to any one of claims 9-12; and
(2) a particle protein component, comprising a nanoparticle protein, wherein
the antigenic component and the particle protein component are subjected to a covalent binding reaction to form the immunogenic complex;
preferably, the antigenic component further comprises a binding peptide 1, and the particle protein component further comprises a binding peptide 2; the G protein mutant and the binding peptide 1 form a fusion protein, and the nanoparticle protein and the binding peptide 2 form a fusion protein; the antigenic component and the particle protein component are covalently bound to each other via the binding peptide 1 and the binding peptide 2 to form the immunogenic complex; the G protein mutant and the binding peptide 1 form a fusion protein, and the fusion protein is assembled into the trimer or the multimer;
preferably, the binding peptide 1 comprises the amino acid sequence set forth in SEQ ID NO: 19, and the binding peptide 2 comprises the amino acid sequence set forth in SEQ ID NO: 20.

16. The immunogenic complex according to claim 15, wherein the antigenic component further comprises a linker peptide 1, and the particle protein component further comprises a linker peptide 2; the antigenic component is formed by fusing the G protein mutant, at the C-terminus, with the binding peptide 1 via the linker peptide 1; the particle protein component is formed by fusing the nanoparticle protein, at the N-terminus, with the binding peptide 2 via the linker peptide 2; the trimer or multimer is formed by self-assembly after fusing the G protein mutant, at the C-terminus, with the binding peptide 1 via the linker peptide 1;
optionally, the linker peptide 1 is selected from the amino acid sequences of (GGGGS)ₙ, (EAAAK)ₙ, (GSGGSG)ₙ, (GGS)ₙ, or (GSG)ₙ, wherein n may be an integer greater than 0 and less than or equal to 5; the linker peptide 2 is selected from the amino acid sequences of (GGS)ₙ, (GGGGS)ₙ, (EAAAK)ₙ, or (GSGGSG)ₙ, wherein n may be an integer greater than 0 and less than or equal to 10;
optionally, both the antigenic component and the particle protein component comprise a histidine tag.

17. The immunogenic complex according to claim 15 or 16, wherein the amino acid sequence of the G protein mutant is set forth in any one of SEQ ID NOs: 2-9; preferably, the amino acid sequence of the G protein mutant is set forth in any one of SEQ ID NOs: 2 and 4-9.

18. The immunogenic complex according to any one of claims 15-17, wherein the nanoparticle protein is NPM, AP205, or ferritin protein; preferably, the amino acid sequence of NPM is set forth in SEQ ID NO: 21.

19. A method for preparing an immunogenic complex, comprising:
(1) separately ligating coding genes of the antigenic component and the particle protein component as defined in any one of claims 15-18 into expression vectors to construct recombinant expression plasmids;
(2) constructing a recombinant host cell capable of expressing the antigenic component and the particle protein component in the host cell;
(3) expressing fusion proteins using the recombinant host cell and purifying the recombinant fusion proteins; and
(4) subjecting the antigenic component and the particle protein component to a covalent binding reaction, so as to obtain the immunogenic complex.

20. The method according to claim 19, wherein in step (1), the plasmid expressing the antigenic component is selected from pcDNA3.4, and the vector expressing the particle protein may be selected from pET-28a(+) and pET-30a(+);
optionally, in step (2), the host cell expressing the antigenic component is CHO, and the host cell expressing the particle protein is *Escherichia coli*.

21. An immune composition, comprising the rabies virus G protein mutant according to any one of claims 1-8, or the rabies virus G protein trimer or multimer according to any one of claims 9-12, or the immunogenic complex according to any one of claims 15-18, wherein optionally, the immune composition of the present disclosure further comprises a pharmaceutically acceptable carrier.

22. The immune composition according to claim 21, wherein the pharmaceutically acceptable carrier comprises a stabilizer, an excipient, a surfactant, a buffering agent, and a pH regulator;
the stabilizer is preferably sucrose or arginine, the excipient is preferably mannitol, the surfactant is preferably Tween-80, the buffering agent is preferably disodium hydrogen phosphate dihydrate or sodium dihydrogen phosphate dihydrate, and the pH regulator is preferably hydrochloric acid.

23. A rabies vaccine, comprising the immune composition according to claim 21 or 22 and an adjuvant, wherein the adjuvant enhances the immune response through a number of mechanisms, including: lymphocyte recruitment, stimulation of B and/or T cells, and stimulation of macrophages, and the vaccine includes a human vaccine or a veterinary vaccine.

24. The vaccine according to claim 23, wherein the adjuvant is selected from: at least one of an aqueous adjuvant, a mineral adjuvant, an oil-based emulsion adjuvant, a microbial adjuvant, a liposome adjuvant, a cytokine adjuvant, a nucleic acid adjuvant, a Chinese herbal adjuvant, and a chemical adjuvant.

25. The vaccine according to claim 23, wherein the adjuvant is selected from a Toll-like receptor agonist, a complex adjuvant, a nano-adjuvant, a trehalose synthetic derivative, a genetically engineered toxoid, and a biodegradable polymeric microsphere; preferably, the adjuvant is selected from poly(I:C), GLA-LSQ, flagellin, CpG1018, AS01, AS03, AS04, Covaxin, AS37, PIKACA, BC adjuvant system, SWE, and MF59.

26. The vaccine according to claim 23, wherein the adjuvant comprises the following components (w/w):
4.2% of squalene, 0.5% of Span 85, 0.5% of Tween-80, 0.264% of citric acid, and 0.016% of sodium citrate.

27. A kit, comprising the vaccine according to any one of claims 23-26, and a device and a container required for administering the vaccine, including: needle/syringe devices, powder containers, and solvent containers.

28. Use of the rabies virus G protein mutant according to any one of claims 1-8, the rabies virus G protein trimer or multimer according to any one of claims 9-13, the immunogenic complex according to any one of claims 15-18, the immune composition according to claim 21 or 22, or the vaccine according to any one of claims 23-26, or the kit according to claim 27 in preparing a medicament for preventing or treating a related disease caused by rabies virus infection, preferably in preparing a medicament for pre-exposure prophylaxis and post-exposure prophylaxis of rabies virus infection.
